# NEW EUROPEAN PATENT SPECIFICATION

(11) **EP 0 120 658 B2**
(45) Date of publication and mention of the opposition decision: **02.09.1998**
(45) Mention of the grant of the patent: 16.01.1991
(21) Application number: 84301800.3
(22) Date of filing: 16.03.1984
(51) Int. Cl.: C12Q 1/68

(54) **Method for identifying and characterizing organisms**
Verfahren zur Identifizierung und Charakterisierung von Organismen
Méthode pour l'identification et la caractérisation d'organismes

(30) Priority: 21.03.1983 US 477431
(43) Date of publication of application: 03.10.1984
(73) Proprietor: Webster, John A., Jr., West Chester Pennsylvania 19382 (US)
(72) Inventor: Webster, John A., Jr., West Chester Pennsylvania 19382 (US)
(74) Representative: Sheard, Andrew Gregory

(56) References cited:
- WO-A-83/01073
- US-A- 3 930 956
- US-A- 4 358 535

## Description

The present invention relates to a method for rapidly and accurately establishing the species of organisms, including prokaryotic and eukaryotic organisms, such as bacteria, plants, and animals.

The classification of living organisms has traditionally been done along more or less arbitrary and somewhat anificial lines. For example, the living world has been divided into two kingdoms: *Plantae* (plants) and *Animalia* (animals). This classification works well for generally familiar organisms, but becomes difficult for such organisms as unicellular ones (e.g., green flagellates, bacteria, blue-green algae), since these differ in fundamental ways from the "plants" and "animals".

It has been suggested to simply divide organisms with respect to the internal architecture of the cell. In this scheme. all cellular organisms are either prokaryotic or eukaryotic. Prokaryotes are less complex than eukaryotes, they lack internal compartmentalization by unit membrane systems and lack a defined nucleus. Prokaryotic genetic information is carried in the cytoplasm on double-stranded, circular DNA; no other DNA is present in cells (except for the possible presence of phage, bacterial viruses, and circular DNA plasmids, capable of autonomous replication). Eukaryotes, on the other hand, have a multiplicity of unit membrane systems which serve to segregate many of the functional components into specialized and isolated regions. For example, genetic information (DNA) can be found in a well-compartmentalized nucleus and also in organelles: mitochondria and (in photosynthetic organisms) chloroplasts. The replication, transcription, and translation of the eukaryotic genome occurs at either two or three distinct sites within the cell: in the nucleocytoplasmic region, in the mitochondrion, and in the chloroplast.

The differences between prokaryotes and eukaryotes, however, breaks down when a comparison of mitochondria and chloroplasts is carried out with prokaryotes: these organelles are today considered to have been derived from free-living prokaryotes, which entered into an endosymbiotic relation with primitive eukaryotes, and eventually became closely integrated with the machinery of the host cell and incapable of independent existence (see e.g., *Fox, G. E. et al, Science 209*: 457-463 (1980), at 462; *Stanier, R. Y. et al,* "The Microbial World," Fourth Edition, Prentice-Hall, Inc., 1976, at p. 86). For example, it has been demonstrated that DNA from mouse L cell mitochondria carrying the ribosomal RNA gene region exhibits notable sequence homologies to *Escherichia coli* ribosomal RNA, thus providing strong support for the endosymbiotic model (*Van Etten, R. A. et al, Cell. 22*: 157-170 (1980)). It has also been shown that the nucleotide sequence of 23S ribosomal DNA with *Zea mays* chloroplast has 71% homology with 23S ribosomal DNA from *E. coli (Edwards, K. and Kossel, H., Nucleic Acids Research, 9*: 2853-2869 (1981)); other related work *(Bonen, L. and Gray, M. W., ibid,* 8: 319-335 (1980)) also further supports the general concept.

In this model the eukaryotic cell is a phylogenetic "chimera" with organelle components that are clearly prokaryotic in nature. The "prokaryotic-eukaryotic" dichotomy then, also has drawbacks, even as a broad classification method.

Where classification of organisms becomes more than a specific exercise is in the identification of plants and animals for hybridization and breeding purposes, and in the accurate and reliable identification of microorganisms which may infect so-called "higher" organisms or other media. For example, the plant-breeder, cattle breeder, or fish breeder may wish to have a quick and reliable means of identifying different species of their subjects. The veterinarian, physician, or horticulturist may wish to have an accurate identification of any infectious organisms (parasites, fungi, bacteria, etc.) and viruses present in examined plant or animal tissues. The correct identification of species of these organisms and viruses is of panicular imponance.

The problem can best be illustrated by referring to the identification of bacteria. Names of bacterial species usually represent many strains, and a strain is considered to be a population derived from a single cell. Bacterial species are usually defined by describing the degree of homogeneity and diversity of attributes in representative samples of strains of species. Precise definitions of bacterial species are difficult to express because subjective limits to strain diversity within species are required to define species boundaries. *(Buchanan, R. E., International Bulletin of Bacteriological Nomenclature and Taxonomy, 15*: 25-32 (1965)). The practical application of definitions of species to the identification of an unknown bacterial strain requires the selection of relevant probes, such as substrates and conditions to detect phenotypic attributes, and radioactively-labeled DNA from the same species. Because of the diversity of bacterial species, a screening procedure is the primary tool used in the classical, progressive method for identification of a strain. Results of the screening procedure are then used to predict which other laboratory methods and reagents are relevant for definitive identification of the strain. Identification is ultimately based on certain phenotypic and genotypic similarities between the unidentified strain and characterized species. The challenge is to precisely define the boundaries of species, preferably in terms of a standard probe which reveals species-specific information, so that definitions of species can be directly and equally applied to the identification of unknown strains.

*Bergey's Manual of Determinative Bacteriology* (Buchanan, R. E. and Gibbons, N. E., Editors, 1974, 8th Edition, The Williams & Wilkins Company, Baltimore) provides the most comprehensive treatment of bacterial classification particularly for nomenclature, type strains, pertinent literature, and the like. It is, however, only a starting point for the identification of any species since, *inter alia,* it is normally out of date, and is limited in space to describing species quite briefly. (See for example, *Brenner, D. J.*, "Manual of Clinical Microbiology," 3rd Edition, American Society of Microbiology, Washington, D.C., 1980, pages 1-6.)

The term "species", as applied to bacteria, has been defined as a distinct kind of organism, having certain distinguishing features, and as a group of organisms which generally bear a close resemblance to one another in the more essential features of their organization. The problem with these definitions is that they are subjective; *Brenner, supra,* at page 2. Species have also been defined solely on the basis of criteria such as host range, pathogenicity, ability or inability to produce gas in the fermentation of a given sugar, and rapid or delayed fermentation of sugars.

In the 1960's, numerical bacterial taxonomy (also called computer or phenetic taxonomy) became widely used. Numerical taxonomy is based on an examination of as much of the organism's genetic potential as possible. By classifying on the basis of a large number of characteristics, it is possible to form groups of strains with a stated degree of similarity and consider them species. Tests which are valuable for the characterization of one species, however, may not be useful for the next, so this means to define species is not directly and practically applicable to the identification of unknown strains. Although this may be overcome in part by selecting attnbutes which seem to be species specific, when these attributes are used to identify unknown strains, the species definition is applied indirectly. See for example *Brenner, supra,* at pages 2-6. The general method, furthermore, suffers from several problems when it is used as the sole basis for defining a species, among them the number and nature of the tests to be used, whether the tests should be weighted and how, what level of similarity should be chosen to reflect relatedness, whether the same level of similarities is applicable to all groups, etc.

*Hugh, R. H. and Giliardi, G. L.,* "Manual of Clinical Microbiology," 2nd Edition, American Society for Microbiology, Washington, D.C., 1974, pages 250-269, list minimal phenotypic characters as a means to define bacterial species that makes use of fractions of genomes. By studying a large, randomly selected sample of strains of a species, the attributes most highly conserved or common to a vast majority of the strains can be selected to define the species. The use of minimal characters is progressive and begins with a screening procedure to presumptively identify a strain, so that the appropriate additional media can be selected. Then the known conserved attributes of the species are studied with the expectation that the strain will have most of the minimal characters. Some of the minimal characters do not occur in all strains of the species. A related concept is the comparative study of the type, the neo-type, or a recognized reference strain of the species. This control is necessary because media and procedures may differ among laboratories, and it is the strain, not the procedure, that is the standard for the species.

A molecular approach to bacterial classification is to compare two genomes by DNA-DNA reassociation. A genetic definition of species includes the provision that strains of species are 70% or more related. With DNA-DNA reassociation a strain can be identified only if the radioactively labeled DNA probe and unknown DNA are from the same species. The practical application of this 70% species definition however is limited by selection of an appropriate probe. This may be overcome in part by selecting phenotypic attributes which seem to correlate with the reassociation group, but when these are used alone the DNA-DNA reassociation species definition is also applied indirectly.

*Brenner*, *supra*, at page 3, states that the ideal means of identifying bacterial species would be a 'black box' which would separate genes, and instantly compare the nucleic acid sequences in a given strain with a standard pattern for every known species-something akin to mass spectrophotometric analysis.
*Brenner*, however, concedes that although restriction endonuclease analysis can be done to determine common sequences in isolated genes, "we are not at all close to having an appropriate black box, especially one suited for clinical laboratory use." His words could be equally applied to any species of organism.

This brief review of the prior art leads to the conclusion that there presently exists a need for a rapid, accurate, and reliable means for establishing the species of unknown bacteria and other organisms, especially to establish the species of the organism of a disease, or of a desirable biochemical reaction. The method should be generally and readily useful in clinical laboratories, should not be dependent on the number of tests done, on the subject prejudices of the clinician, nor the fortuitous or unfortuitous trial and error methods of the past. Further, a need also exists for a method useful for establishing and distinguishing genera and species of *any* living organism, which can be readily and reliably used by veterinarians, plant-breeders, toxicologists, animal breeders, entomologists and in other related areas, where such identification is necessary.

It is therefore an object of the invention to provide a method which may be found to be quick, reliable and accurate of objectively establishing the species of organisms, especially - but not limited to - microorganisms.

Yet another object of the invention is to provide a method of establishing the species of organisms such as bacteria which utilizes the organisms' genome.

Another object of the invention is to provide a method of establishing species and genera of pathogenic organisms in the clinical laboratory, so as to provide the capability of characterizing and identifying the cause of any given animal or plant disease.

Still another object of the invention is to provide various products useful in the aforementioned methodologies.

These and other objects of the invention, as will hereinafter become more readily apparent, have been attained by providing:

A method of establishing the species of an unknown organism which comprises determining the position of part or whole of an evolutionarily conserved sequence or sequences in genetic material (other than an evolutionarily conserved sequence occurring in ribosomal RNA information-containing nucleic acid) of said organism, relative to a known position of restriction endonuclease cleavage sites in said genetic material, thereby to obtain an identifying genetic characterization of said unknown organism, comparing said characterization with information from at least two sets of identifying genetic characterizations derived from the same conserved sequences, each of said sets defining a known organism species, and establishing the species of said unknown organism.

Still another object of the invention has been attained by providing:

A method of diagnosing a pathogenic organism infection in a sample which comprises identifying the organism in said sample by the aforementioned method.

Preferred embodiments of the invention will now be described.

This invention is based on the inventor's realization that, if species are discrete clusters of strains related to a common speciation event, there should be, despite divergence, a likeness shared by strains that objectively defines the species boundary; strains of species should contain structural information which is a clue to their common origin. The greatest amount of an organism's past history survives in semantides, DNA and RNA, *(Zuckerkandle, E. and Pauling, L., Journal of Theoretical Biology, 8*: 357-366 (1965)).

In European Patent Application (EP-A-) No. 0076123 (herein incorporated by reference) the inventor described a system for the definition of species and characterization of organisms which makes use of information contained in ribosomal RNA genes (rRNA). Ribosomal RNA has a structural and functional role in protein synthesis *(Schaup, Journal of Theoretical Biology, 70*: 215-224 (1978)), and the general conclusion from rRNA-DNA hybridization studies, is that the base sequences of ribosomal RNA genes are less likely to change, or are more conserved during evolution, than are the majority of other genes *(Moore, R. L., Current Topics In Microbiology and Immunobiology,* Vol. *64*: 105-128 (1974), Springer-Verlag, New York). For example, the primary structure of 16S rRNA from a number of bacterial species has been inferred from oligonucleotide analysis *(Fox, G. E. et al, International Journal of Systematic Bacteriology, 27*: 44-57 (1977)). There are negligible differences in the 16S oligomer catalogs of several strains of *E. coli* (*Uchida, T. et al, Journal of Molecular Evolution, 3*: 63-77 (1974)); the substantial differences among species, however, can be used for a scheme of bacterial phylogeny (*Fox, G. E., Science, 209*: 457-463 (1980)). Different strains of a bacterial species are not necessarily identical; restriction enzyme maps show that different EcoR I sites occur in rRNA genes in two strains of *E. coli* (*Boros, I. A. et al, Nucleic Acids Research 6*: 1817-1830 (1979)). Bacteria appear to share conserved rRNA gene sequences and the other sequences are variable (*Fox,* 1977, *supra*).

The present inventor had thus discovered that restriction endonuclease digests of DNA have sets of fragments containing conserved sequences that are similar in strains of a species of organism (e.g., bacteria), but different in strains of other species of the organism; i.e., despite strain variation, enzyme specific sets of restriction fragments with high frequencies of occurrence, minimal genotypic characters, define the species. This is the essence of the invention described in EP-A-0076123 and also that of the invention described herein.

The present invention constitutes an extension of the concepts developed in EP-A-0076123, in that it has further been discovered that there exist sequences, in addition to those of rRNA, which are highly conserved through evolution and which may be as useful as rRNA sequences in the identification system. In other words, the present invention provides means for carrying out the identification and characterization techniques of EP-A-0076123, using any probe which is conserved, other than rRNA. The present invention also provides additional examples of methods which may be used in the identification process. The present inventor has also discovered that the method is general, in that it is applicable to both eukaryotic and prokaryotic DNA, using a conserved nucleic acid probe from any organism, prokaryotic or eukaryotic, of the same or different (classic) taxonomic classification than the organism being identified.

The invention offers an objective method of establishing the species of organisms based on conserved sequences of DNA or other genetic material in relation to restriction endonuclease sites. The detection of restriction fragments containing a conserved sequence may be carried out by hybridizing or reassociating DNA segments with nucleic acid containing conserved sequence information from a probe organism.

By the "organism" whose species can be established by the process of the invention, it is meant to include virtually any organism which, by definition, contains DNA or RNA in its genome. In this respect it is useful to refer to a classical taxonomic scheme as a point of reference.

All organisms belonging to the Kingdoms Monera, Plantae and Animalia are included. For example, among those of the Kingdom Monera can be mentioned the Schizomycetes (Bacteria) of the classes myxobacteria, spirochetes, eubacteria, rickettsiae, and the cyanopytha (blue green algae). Among those of the Kingdom Piantae can be mentioned the Division Euglenophyta (Euglenoids), Division Chlorophyta (green-algae) classes chlorophyceae and charophyceae, Division Chrysophyta, classes xanthophyceae; chrysophyseae, bacillariophyceae; Division Pyrrophyta (Dinoflagellates); Division Phaeophyta (Brown algae); Division Rhodophta (Red algae); Division Myxomycophyta (slime molds), classes myxomycetes, acrasiae, plasmodiophoreae, labynnthuleae; Division Eumycophyta (true fungi), classes phycomycetes, ascomycetes, and basidomycetes; Division Bryophta, classes hepaticae, anthocerotae, and musci; Division Tracheophyta (Vascular plants), subdivisions psilopsida, lycopsyda, sphenopsida, pteropsida, spermopsida classes cycadae, ginkgoae, coniferae, gneteae and angiospermae subclasses dicotyledoneae, monocotyloedoneae. Among those of the Kingdom Animalia can be mentioned the Subkingdom Protozoa, Phylum Protozoa (Acellular animals) subphylum plasmodroma, classes flagellata, sarcodina and sporozoa; subphylum ciliophora, class ciliata; the Subkingdom Parazoa, Phylum porifera (Sponges), class calcarea, hexactinellida, and desmospongiae; the Subkingdom Mesozoa, Phylum mesozoa; the Subkingdom Metazoa, Section Radiata, Phylum coelenterata, classes hydrozoa, scyphozoa, anthozoa, Phylum ctenophora, classes tentaculata and nuda; Section Protostomia Phylum platyhelmintes (flat-worms) classes tubellana, trematoda, and cestoda; Phylum nemertina; Phylum acanthocephala; Phylum aschelmintles, classes rotifera, gastrotricha, kinorhyncha, priapulida, nematoda and nematomorpha; Phylum entoprocta; Phylum ectoprocta, classes gymnolaemata and phylactolaemata; Phylum phoronida; Phylum braciopoda, classes inarticulata and articulata; Phylum mollusca (molluscs) classes amphineura, monoplacophora, gastropoda, scaphopoda, pelecypoda, and cephalopoda; Phylum sipunculida; Phylum echiurida; Phylum annelida, classes polychaeta, oligochaeta and hirudinae; Phylum onychophora; Phylum tardigrada; Phylum pentastomida; Phylum arthropoda, subphylum trylobita, subphylum chelicerata classes xiphosura, arachmida, pycnogomida, subphylum mandibulata classes crustacea, chilopoda, diplopoda, pauropoda, symphyla, insecta of the orders collembola, protura, diplura, thysanura, ephemerida, odonata, orthoptera, dermaptera, embiania, plecoptera, zoraptera, corrodentia, mallophaga, anoplura, thysasnoptera, hemiptera, neuroptera, coleoptera, hymenoptera, mecoptera, siphonaptera, diptera, trichoptera and lepidoptera; those of the Section Deuterostomia, phylum chaetognatha, phylum echinodermata, classes crinoidea, asterordea, ophiuroidea, echinoidea, and holoturoidea, phylum pogonophora; phylum hemichordata, classes enteropneusta, and pterobranchia; phylum chordata, subphylum urochordata, classes ascidiaciae, thaliaceae, larvacea; subphylum cephalochordata, subphylum vertebrata, classes agnatha, chondrichthyes, osteichthyes (subclass saccopteiygii orders crossopterygii and dipnoi), amphibia, repitilia, aves and mammalia, subclass prototheria, subclass theria, orders marsupialia, insectivora, dermoptera, chiroptera, primates, edentata, pholidota, lagomorpha, rodentia, cetaceae, carnivora, tubulidentata, probosicdea, hyracoidea, sirenia, perissodactyla and artiodactyla.

It is understood that beyond the order, the organisms are still classified according to their families, tribes, genus and species. In addition, cell cultures (plant or animal), as well as viruses can also be identified. These classifications are used in this application for illustrative purposes only, and are not to be taken as exclusive. The organism is either known or unknown, most commonly the organism is an unknown being identified.

Functionally, for the purposes of this invention, it is convenient to divide all organisms into the eukaryotes and the prokaryotes. When identifying a prokaryotic organism, the DNA to be analyzed is that present in the cells or in the non-compartmentalized chromosomes. When identifying a eukaryotic organism one may either use the nuclear DNA or the organelle DNA (mitochondrial DNA or chloroplast DNA).

Briefly, high molecular weight DNA and/or small circular DNAs are isolated from the organism to be identified in order to analyze the conserved sequences. The DNA's are extracted by methods which are well-known to the art.

The DNA's are analyzed to ascertain both 1) the presence and position of the conserved sequences and 2) their position relative to endonuclease restriction sites. The easiest way to analyze for the presence of the conserved sequences is to utilize a polynucleotide probe capable of hybridizing with the conserved DNA sequence. However, direct sequence information as obtained by chemical sequence determination and analysis thereof could also be utilized. In EP-A-0076123 the probe utilized was an rRNA information containing-probe; in this case any other probe having conserved sequences could be used. In an analogous manner, the easiest way of finding a given set of endonuclease restriction sites is to cleave the DNA with the appropriate restriction enzymes. (This, indeed, is the manner taught and practised in EP-A-0076123.) However, alternative methods, such as sequence information coupled with known restriction site sequences, or cleavage and partial sequencing could also be used.

Most commonly DNA's are going to be cut at specific sites into fragments by restriction endonucleases. The fragments are separated according to size by a chromatographic system. In EP-A-0076123 gel chromatography was used as an example of a useful chromatographic system. However, other systems can also be used, such as high pressure liquid chromatography, capillary zone electrophoresis, or other separation techniques. In using gel chromatography, the fragments are separated, the gels are stained, as is otherwise well-known in the art, and standardized as to the fragment sizes using standard curves constructed with fragments of known sizes. The separated fragments may then be transferred to cellulose nitrite paper by the Southern blot technique (*Southern, E. M., Journal of Molecular Biology, 38*: 503-517 (1975), herein incorporated by reference), and covalently bound thereto by heating. The fragments containing the conserved sequences are then located by their capacity to hybridize with a nucleic acid probe containing conserved sequence information. Alternatively, hybridization can occur after digestion but before separation; or restriction cleavage can occur after hybridization, followed by separation of the fragments.

The nucleic acid probe can either be non-radioactively labeled or, preferably, radioactively labeled. When radioactively labeled, the probe can be RNA, or preferably DNA which is complementary to RNA (cDNA), either synthesized by reverse transcription or contained on a cloned-fragment, which can be labeled, for example, by nick translation. Also, synthetic oligodeoxyribonucleotides may be prepared with labeled nucleotides.

The well-defined probe is derived from an arbitrarily chosen organism, see *infra,* or may be a consensus sequence. Once hybridization has occurred, the hybridized fragments are detected by selectively detecting double stranded nucleic acid (non-radiolabeled probe), or visualized by, e.g., autoradiography (radiolabeled probe). The size of each fragment which has been hybridized is relative to the restriction sites and is determined from the distance traveled using standard curves, as described previously. The amount of hybridization, the pattern of hybridization, and the sizes of the hybridized fragments, which are relative to restriction sites, can be used individually or in conjunction to identify the organism.

The genetic characterization that emerges from this technique can be readily compared to equivalent characterizations derived from at least two and up to a multiplicity of known, standard organisms, genera or species. After a preliminary broad classification has already been carried out (using, for example, classical taxonomy), the comparison can be either by visual inspection and matching of appropriate chromatographic patterns, (as in EP-A-0076123) by comparison of hybridized restriction fragment sizes, by band intensity (amount of hybridization) or by any combination thereof. Ideally, the comparison is carried out with a one-dimensional computer-based pattern recognition system, such as those used in point-of-sale transactions.

The present inventor discovered that when using the aforementioned method, the genetic characterizations for organisms of the same species are substantially similar, with minor variations allowed for intraspecies differences due to strain variations, whereas differences between species, and differences between genera (and higher classifications) are maximal.

The "probe organism" used in the present invention, and from which is obtained the nucleic acid probe, can also be any of the aforementioned organisms; it can be either eukaryotic or prokaryotic. The only limitation is given by the fact that the conserved sequence-containing probe should hybridize maximally with the unknown organism's DNA.

There are four types of conserved sequence information-containing probes: 1) prokaryotic probes (especially bacterial-derived), 2) eukaryotic mitochondrial probes, 3) eukaryotic chloroplast probes, and 4) eukaryotic non-organelle probes. There are also four sources of DNA (to be endonuclease digested): 1) prokaryotic cellular DNA, 2) eukaryotic mitochondrial DNA, 3) eukaryotic chloroplast DNA, and 4) eukaryotic nuclear DNA. The following hybridization table can thus be constructed (Table 1).

**TABLE 1**

| Hybridization Table | | | | |
|---|---|---|---|---|
| | | Conserved Gene Sequence Probe | | |
| Unknown organism DNA | Prokaryotic | | Eukaryotic | |
| | | Mitochondrial | Chloroplast | Nonorganelle |
| Prokaryotic | + | + | + | - |
| Eu.⁽¹⁾ Mitochondria | + | + | + | - |
| Eu. Chloroplast | + | + | + | - |
| Eu. Nuclear | -⁽²⁾ | - | - | + |

| | | | | |
|---|---|---|---|---|
| (1) Eu = Eukaryotic | | | | |
| (2) = refers to generally less effective hybridization, see Example 4, *infra*. | | | | |

The Table shows which probes can generally be maximally hybridized with which unknown DNA. For example, one can identify a eukaryotic organism by extracting species specific mitochondrial or chloroplast DNA, endonuclease-digesting it and hybridizing the digest with either a prokaryotic probe, or with an organelle derived eukaryotic probe. In the same manner, one can identify a prokaryotic organism by extracting species-specific cellular DNA, endonuclease-digesting it, and hybridizing the digest with either a prokaryotic probe, or an organelle-derived eukaryotic RNA probe. Also, one can identify a eukaryotic organism by extracting and digesting species-specific nuclear DNA, and hybridizing it with a non-organelle derived eukaryotic probe. Eukaryotes could be defined by one or any combination of the nuclear, mitochondria, or in some cases chloroplast systems. These cross-hybridizations are based on the fact that nucleic acid derived from eukaryotic organelles has extensive homology with evolutionarily conserved sequences from prokaryotic nucleic acid, but that the same homologies are generally not present to such extent between nuclear-derived eukaryotic DNA and prokaryotic DNA.

The choice of any pair of DNA to be digested and accompanying probe is arbitrary, and will depend on the organism being identified, i.e. it will depend on the question asked. For example, in detecting a prokaryotic species (e.g. bacteria) present in or together with a eukaryotic cell (e.g. animal or plant) for purposes of detecting and identifying an infecting agent, one may choose a prokaryotic probe and work under conditions where organelle-derived DNA is not extracted or only minimally extracted. In this manner one assures that interference between organelle-derived DNA and prokaryotic DNA is minimal. In identifying a eukaryotic species (which is not infected with a prokaryote) with a prokaryotic probe, it is best to maximize the concentration of organelle-derived DNA, as for example by separating organelles from nuclei, and then extracting only organelle DNA. If one wishes to identify a eukaryotic organism which has been infected with a prokaryotic organism, it is best to use a non-organelle, non-prokaryotic derived probe since it will generally not hybridize well with the DNA from the prokaryote.

It is preferred to use a pair (DNA and probe) from the same kingdom, or same subkingdom, or same section, or same phylum, or same subphylum, or same class, or same subclass, or same order, or same family or same tribe or same genus. It is particularly preferred to use prokaryotic probe (e.g. bacterial probe) to hybridize with prokaryotic DNA. In this manner one could detect, quantify, and identify genera, and species of prokaryotic organisms. One of the most preferred prokaryotic probes is derived from bacteria, and further, because of the ease and availability, from *E. coli.* The probe from *E. coli* can be used to establish the species of any organism, especially any prokaryotic organism. Another particularly preferred embodiment is to use eukaryotic probe derived from a given class to identify eukaryotic organisms of the same class (e.g. mammalian probe to identify mammalian organism). Most preferred is to use probe and DNA from the same subclass and/or order and/or family of organisms, (e.g. if identifying a species of mouse, it is preferred to use mouse-derived probe).

The most sensitive and useful pair systems are those where there is less evolutionary distance or diversity between the source of the probe and the unknown DNA.

The phrase "evolutionary conserved genetic material sequence" is used in this invention to denote genetic material, e.g. DNA, sequences that show homology between at least two different species of plants, animals or microorganisms. The homology between two conserved sequences is to be such that, if one of such DNA molecules were to be detectably labelled, sufficient hybridization or annealing would occur if both single stranded DNA molecules or fragments thereof were placed together under hybridization conditions, thereby to produce a duplex of sufficient stability to be detectable by standard methodology (i.e., radiolabelling, enzyme labelling, and the like).

In EP-A-0076123 the evolutionarily conserved sequence exemplified was that of ribosomal RNA genes. This is still a highly preferred gene sequence. However, it has been discovered that other gene sequences exist which are sufficiently conserved across the evolutionary span to be useful.

Examples of such additional sequences are those of genes or portions thereof coding for transfer RNA, or proteins denoted as belonging to the same Superfamily, or same Family, preferably same Subfamily or even same entry in Dayhoff's "Atlas of Protein Sequence and Structure", Volume 5, Supplement 3, 1978, NBR, 1979, pages 9-24, herein incorporated by reference. A Family of proteins is one wherein any two proteins differ from each other by less than 50% amino acid residues in their sequence. A Subfamily of proteins is one wherein any two proteins differ from each other by less than 20% amino acid residues in their sequence. An "Entry" is one wherein any two proteins differ from each other by less than 5% amino acid residues in their sequence.

Specific examples of gene sequences or appropriate portions thereof which can be used are: cytochrome C related genes, cytochrome C, related genes, cytochrome c₁ related, cytochrome b₅ related, ferrodoxin related, rebredoxin related, flavodoxin related, alcohol dehydrogenase related, lactase dehydrogenase related, peroxidase related, adenylase kinase related, phospholipase A₂ related, tryptophan operon related, carboxypeptidase related, subtilisin related, penicillinase related, protease inhibitor related, somatotropin related, corticotropin related, lipotropin related, glucagon related, snake venom toxin related, plant toxin related, antibacterial toxin related, immunoglobulin related gene, ribosomal other than rRNA-related genes, heme carrier genes, chromosomal protein genes, fibrous protein genes, and the like.

The conservation of some of these additional DNA sequences is not as widespread throughout the animal, plant or microbiological domains as is that of the rRNA genes. (Thus the still preferred use of rRNA.) This, however, does not constitute a serious impediment to their use since it may be possible to utilize such additional sequences to identify or characterize organisms within more limited ranges or subdomains. For example it may be posslble to utilize trp D gene sequences from bacteria to generate a trp D bacterial probe and then use this probe to test within the bacterial domain. In fact, it may be possible to use a trp D probe within an even narrower domain (e.g., test for the presence of *Enterobacteriaceae*, or of *Bacillus*, etc.) with a trp D probe from the same order, family or genus. Thus, while the range of applicability of some of the additional probe sequences may not be as broad as that of rRNA probes, their applicability will nevertheless be quite effective within narrower domains.

A probe containing the conserved DNA sequence information is prepared in the same manner as the preparation of rRNA information containing probe exemplified in EP-A-0076123. The probe can thus be RNA, DNA or cDNA, and the like.

The individual steps involved in the technique will be described hereinafter broadly with reference to both eukaryotic and prokaryotic cells when applicable, or specifically for each type of cell if some difference in technique exists.

The first step is extraction of the DNA from the unknown organisms. Nuclear DNA from eukaryotic cells can be selectively extracted by standard methodology well-known to the art (see for example, *Drohan,* W. *et al, Biochem. Biophys Acta*, 521 (1978), 1-15, herein incorporated by reference). Because organelle DNA is small and circular, spooling techniques serve to separate the non circular nuclear DNA from the circular, organelle-derived DNA. As a corollary, the non-spooled material contains the organelle-derived DNA which can separately be isolated by density gradient centrifugation. Alternatively, mitochondria (or chloroplasts) are separated from a mixture of disrupted cells; the purified mitochondrial (or chloroplast) fraction is used for the preparation of organelle-derived DNA while the purified nuclear fraction is used to prepare nuclear DNA. (See for example *Bonen, L. and Gray, M. W., Nucleic Acids Research, 8*: 319-335 (1980).)

Prokaryotic DNA extraction is also well-known in the art. Thus, for example, an unknown bacterium present in any medium, such as an industrial fermentation suspension, agar medium, plant or animal tissue or sample or the like, is treated under well-known conditions designed to extract high molecular weight DNA therefrom. For example, cells of the unknown organism can be suspended in extraction buffer, lysozyme added thereto, and the suspension incubated. Cell disruption can be further accelerated by addition of detergents, and/or by increase in temperature. Protease digestion followed by chloroform/ phenol extraction and ethanol precipitation can be used to finalize the extraction of DNA. An alternative method of extraction, which is much faster than phenol/chlorofonm extraction, is rapid isolation of DNA using ethanol precipitation. This method is preferably used to isolate DNA directly from colonies or small, liquid cultures. The method is described in *Davis, R. W. et al:* "A Manual for Genetic Engineering, Advanced Bacterial Genetics," (hereinafter "Davis"), Cold Spring Harbor Laboratory, Cold Spring Harbor, New York, 1980, pp. 120-121, herein incorporated by reference.

The DNA (prokaryotic or eukaryotic (nuclear or non-nuclear)) is dissolved in physiological buffer for the next step. There are a variety of possible steps to be followed after isolation of the desired DNA. One of these steps is endonuclease digestion.

Digestion of extracted DNA is carried out with restriction endonuclease enzymes. Any restriction endonuclease enzyme can be used. Preferably it is not from the same organism species as that being identified, since otherwise, the DNA may remain intact. (This may, in any event, identify the organism, since the enzymes are not expected to cut DNA from the species of their origin.) Since the organism species being characterized may be unknown, obtaining a suitable digest of fragments may entail a minimum amount of trial and error, which can routinely be carried out by those skilled in the art without undue experimentation. Examples of possible restriction endonuclease enzymes are Bgl I, BamH I, EcoR I, Pst I, Hind III, Bal I, Hga I, Sal I, Xba I, Sac I, Sst I, Bcl I, Xho I, Kpn I, Pvu II, Sau IIIa, or the like. See also *Davis, supra*, at pp. 228-230, herein incorporated by reference. A mixture of one or more endonucleases can also be used for the digestion. Normally, DNA and endonuclease are incubated together in an appropriate buffer for an appropriate period of time (ranging from 1 to 48 hours, at temperatures ranging from 25°C-65°C, preferably 37°C).

The resulting identifying genetic characterization will depend on the type or types of endonucleases utilized, and will be endonuclease-specific. It is therefore necessary to note which enzyme or enzymes have been used for the digestion since comparative characterizations used in a catalog should have been prepared using the same enzyme or enzymes.

An alternative step is to define endonuclease sites on the desired DNA molecules without digestion thereof, for example, by sequencing and reference to a restriction site library. Obviously, digestion is the more efficient method of noting such sites, but the method need not be limited thereto. The essence of the invention is the discovery that the position of conserved sequences along DNA, relative to the position of endonuclease restriction sites, forms a set which is characteristic for each species. Thus, any technique which yields the desired information (position of the genes vis a vis position of the sites) will be useful in the invention.

Also, the position of the conserved sequences along the DNA molecule is best noted by use of a hybridization probe. This probe is allowed to anneal to restriction fragments of the unknown's DNA. However, any other method that would allow the determination of the conserved DNA sequences, such as sequencing, would also be useful. When using the hybridization probe it is preferred to first digest and separate DNA fragments according to size, and then to hybridize the separated fragments. However, it is possible to first digest and anneal DNA with a molar excess of probe and/or sequences complementary to probe and then separate the mixture. For example, unknown DNA can be digested with a restriction endonuclease, denatured, and hybridized in liquid with a molar excess of one or more small detectably labeled DNA fragments or synthetic oligodeoxyribonucleotides complementary to a portion or portions of the conserved sequence of interest. Since most restriction enzymes cut fairly infrequently in DNA, in most cases the double-stranded region or regions of the hybrid will be small relative to the size of the restriction fragment. The hybridization reaction is conducted under conditions where only the oligodeoxyribonucleotides hybridize. The unreacted, single-stranded DNA fragments, and the DNA fragments containing the hybridized oligodeoxyribonucleotides are separated by conventional chromatographic techniques. The labeled DNA fragments will appear in predictibly sized fractions. It is also possible to first anneal DNA with a molar excess of probe, then digest, and then separate the mixture. When the solution is incubated for a short time period or to a low cₒt, the restriction sites will be limited to the hybridized, double-stranded regions. When the solution is incubated for a long time period or to a high Cₒt, the unknown DNA will anneal, thus creating a labeled duplex susceptible to restriction endonuclease cleavage. Unreassociated single-stranded tails may be removed with a nuclease such as S1. Unpaired bases may be filled in using DNA polymerase I or T4 polymerase.

Alternatively, one could find subsequences within the conserved sequence information (e.g. 20-, 30-, or 50-mers), which are more highly conserved than the remainder of the conserved region chosen as the probe. Those "shorter" sequences can be made synthetically or enzymatically, if desired, and may incorporate labeled nucleotides. Single-stranded, predigested DNA from the unknown is allowed to incubate with these shorter, highly conserved fragments and allowed to hybridize thereto. Separation would then be carried out on the digest mixture containing fragments partly annealed to the shorter labeled probes. (Thus, separation would occur after hybridization.) Separation could be by liquid chromatography, since the digest mixture would for all practical purposes behave as a mixture of essentially single-stranded fragments.

As indicated, a preferred method is to first digest, then separate, and then hybridize. Thus, after endonuclease digestion, the incubation mixture, which contains fragments of varying sizes, is preferably separated thereinto by an appropriate chromatographic method. Any method which is capable of separating nucleic acid digests according to size, and which allows the eventual hybridization with the nucleic acid probe when hybridization is the last step, can be used. For example, gel electrophoresis, high pressure liquid chromatography or capillary zone electrophoresis can be used. (Jorgenson, J. W., J. of HRC and CC, 4: 230-231 (1981).) Presently preferred is gel electrophoresis, most preferred is agarose gel electrophoresis. In this system, the DNA digests are normally electrophoresed in an appropriate buffer, the gels are normally immersed in an ethidium bromide solution, and placed on a UV-light box to visualize standard marker fragments which may have been added. Detectably labeled standard marker fragments may be used as well.

After separation and visualization, the DNA fragments are transferred onto nitrocellulose filter paper or onto charge-modified nylon membranes by the method of *Southern* (Journal of Molecular Biology, 38: 503-517 (1975)). The transfer can be carried out after denaturation and neutralization steps, and is usually done for long periods of time (approximately 10-20 hours) or, alternatively by means of an electrically driven transfer from gel to paper. Instruments used to accelerate the transfer from gel to paper are commercially available. The receiving nitrocellulose filter papers are then normally baked at high temperatures (60-80°C) for several hours, to bind the DNA to the filter.

Alternatively, transfer can be avoided by using the recent method of direct hybridization of *Purrello, M. et al,* Anal. Biochem., 128: 393-397 (1983).

The probe utilized for the hybridization of the paper-bound DNA digest fragments is a defined nucleic acid probe preferably from or derived from a given well-defined organism or the base sequence is known.

Alternatively, the probe sequence may not have a natural counterpart; i.e., it may be a consensus sequence with a base at each position that is most commonly present at that residue in a number of equivalent sequences in different species. The consensus sequence is then generally able to form a more stable hybrid than any one of the naturally occurring sequences. The probe may be a synthetic oligodeoxyribonucleotide molecule made by covalently attaching individual nucleotides in a predetermined sequence. Synthetic molecules may be prepared, for example, by the triphosphate method of synthesis (Alvarado-Urbina *et al*, Science *214*: 270-274 (1981)). The probe molecules may be of any useful size, and more than one sequence may be in the probe solution. For example, several 20 base sequences might be used to detect several highly conserved regions in rRNA genes. It may be detectably labeled or non-labeled, preferably detectably labeled. In such case, it is either detectably labeled RNA, but preferably nick-translated labeled DNA, cloned DNA, or detectably labeled DNA which is complementary to the RNA from the probe organism (cDNA), all of which contain highly conserved DNA sequence information. Synthetic oligodeoxyribonucleotides may be prepared with detectably labeled nucleotides, so the molecule is labeled by incorporating labeled nucleotide residues. Depending on the choice of pair, the probe may be from a prokaryote, or from a eukaryote (cytoplasm-derived, or organelle derived). Most preferably, the detectable label is a radioactive label such as radioactive phosphorus (e.g., ³²P,³H or ¹⁴C) or a biotin-avidin-based system. The nucleic acid probe may also be labeled with metal atoms. For example, uridine and cytidine nucleotides can form covalent mercury derivatives. Mercurated nucleoside triphosphates are good substrates for many nucleic acid polymerases, including reverse transcriptase (*Dale et al, Proceedings of the National Academy of Sciences 70*: 2238-2242, 1973). Direct covalent mercuration of natural nucleic acids has been described (*Dale et al, Biochemistry 14*: 2447-2457). Reannealing properties of mercurated polymers resemble those of the corresponding nonmercurated polymers (*Dale and Ward, Biochemistry 14*: 2458-2469). Metal labelled probes can be detected, for example, by photo-acoustic spectroscopy, x-ray spectroscopy, e.g., x-ray fluorescence, x-ray absorbance, or photon spectroscopy.

The isolation and preparation of any desired conserved DNA sequence-containing probe is within the skill of the art.

If radioactivity labeled probe is used, the same is isolated from the probe organism after growth or cultivation of the organism with nutrients or in culture media containing appropriately radioactive compounds. When the probe is complementary DNA (cDNA), the same is prepared by reverse transcribing isolated RNA from the probe organism, in the presence of radioactive nucleoside triphosphates (e.g., ³²P-nucleosides or ³H-nucleosides).

The labeled probe may also be a nick-translated DNA molecule, especially one obtained from organelle-derived whole circular DNA. In this embodiment, chloroplast or mitochondrial DNA is nick-translated in the presence of radiolabel, and a labeled DNA probe is thereby obtained. The chloroplast labeled probe will hybridize best with chloroplast DNA, and the mitochondrial labeled probe will hybridize best with mitochondrial DNA. The chloroplast (or mitochondrial) nick-translated labeled probe will hybridize second best with mitochondrial (or chloroplast) DNA; it will also hybridize, albeit generally in less favorable fashion, with whole plant (or animal) DNA. The probe may also be obtained from eukaryotic nuclear DNA by nick-translation, although practical considerations would rule against this mode. A more useful approach in this embodiment is to cut out the highly conserved genes from the nuclear eukaryotic DNA (by restriction enzymes), separate the fragments, identify the gene sequences (as by hybridization), and isolate said gene sequences (as by electrophoresis). The isolated sequences may then be recombined into a plasmid or other vector, and after transformation of an appropriate host, cloned in ³²P-containing media. Alternatively, the transformed host is grown, and the DNA is then isolated and labeled by nick-translation; or the DNA is isolated, the sequences are cut out and then labeled. The resulting ribosomal probe will hybridize in the same instances as cDNA (see *infra*).

The preferred nucleic acid probe is radioactively labeled DNA complementary to RNA from the probe organism. The RNA is usually messenger RNA coding for a conserved gene and is substantially free of other RNA's such as transfer RNA (tRNA) or ribosomal RNA (rRNA).

The pure RNA, substantially free of other types of RNA, is incubated with any reverse transcriptase capable of reverse transcribing it into cDNA, preferably with reverse transcriptase from avian myeloblastosis virus (AMV) in the presence of a primer such as calf thymus DNA hydrolysate. The mixture should contain appropriate deoxynucleoside triphosphates, wherein at least one of said nucleosides is radioactively labeled, for example with ³²P. For example, deoxycytidine 5'-(³²P), deoxythymidine 5'-(³²P), deoxyadenine 5'-(³²P), or deoxyguanidine 5'-(³²P) triphosphates can be used as the radioactive nucleosides. After incubation, from 30 minutes to 5 hours at 25°C-40°C, extraction with chloroform and phenol, and centrifugation as well as chromatography, the radioactively labeled fractions are pooled, and constitute the cDNA probe. The radioactively labeled cDNA probe containing conserved DNA information in substantially purified form, i.e., free of non-labeled molecules, free of cDNA which is complementary to other types of RNA, free of proteinaceous materials as well as free of cellular components such as membranes, organelles and the like, also constitutes an aspect of the present invention. A preferred probe is prokaryotic labelled cDNA, most preferred being the bacterial labelled cDNA. The probe species can be any bacterial microorganism, such as those of the family *Enterobacteriaceae, Brucella, Bacillus, Pseudomonas, Lactobacillus, Elaemophilus, Mycobacterium, Vibrio, Neisseria, Bactroides* and other anaerobic groups, *Legionella,* and the like. Although the prokaryotic examples in the present application are limited to the use of *E. coli* as a bacterial prokaryotic probe organism, this aspect of the invention is by no means limited to this microorganism. The use of cDNA in radioactively labeled form as the probe is preferred to the use of radioactively labeled RNA because DNA has greater stability during hybridization.

It is important to recognize that the labeled cDNA probe should be a faithful copy of the RNA, i.e. be one wherein all nucleotide sequences of the template RNA are transcribed each time the synthesis is carried out. The use of a primer is essential in this respect. That the cDNA is a faithful copy can be demonstrated by the fact that it should have two properties following hybridization:
1. The cDNA should protect 100% of labeled RNA from ribonuclease digestion; and
2. The labeled cDNA should specifically anneal to the RNA as shown by resistance to S1 nuclease.

*Beljanski, M. M. et al,* C.R. Acad. Sc Paris t 286, Serie D. p. 1825-1828 (1978), described ³H radioactively labeled cDNA derived from *E. coli* rRNA. The cDNA in this work was not prepared with reverse transcriptase in the presence of a primer as in the present invention, but was prepared with a DNA polymerase I, using as a template rRNA which had been pre-cleaved using ribonuclease U₂. The rRNA digestion product (with RNAse U₂) of *Beljanski et al* has a different base ratio from the initial rRNA, indicating a loss of bases and/or loss of short fragments. Thus the cDNA obtained therefrom is not a faithful copy. In addition, the use of DNA polymerase I used by *Beljanski is* known to favor predominance of homopolymeric over heteropolymeric transcription of rRNA (see *Sarin, P. S. et al, Biochem. Biophys. Res. Comm., 59*: 202-214 (1974)).

In sum, the probe can be seen as being derived al from genome DNA containing conserved sequences, e.g. genes, by cloning and/or nick-translation, b) from RNA itself or c) from cDNA by reverse transcription of RNA.

Normally, the next step in the process of the invention is the hybridization of the separated DNA digest from the unknown organism with the unlabeled or (preferably) radioactively labeled RNA or DNA probe. Hybridization is carried out by contacting the paper containing covalently labeled DNA digest from the unknown, with a hybridization mix containing the probe. Incubation is carried out at elevated temperatures (50-70°C) for long periods of time, filter papers are then washed to remove unbound radioactivity (if needed), air dried and readied for detection. An alternative, highly preferred hybridization, which is much more rapid than the one described above, is the room temperature phenol emulsion reassociation technique of *Kohne, D. E. et al*, *Biochemistry,* 16: 5329-5341 (1977)), which is herein incorporated by reference.

After hybridization, the technique requires selective detection of the appropriately hybridized fragments. This detection can be carried out by taking advantage of the double strandedness of the hybridized fragments and using a selective method therefor (for non-labeled probe), or by autoradiography or by an appropriate radiation scanner which may or may not be computerized, and which may increase the speed of detection (for labeled probe). These techniques are well known to those skilled in the art and will not be further described at this point.

The end product of the technique is an identifying genetic characterization, such as a chromatographic band pattern having peaks and troughs, or preferably, light and dark regions of various intensities, at specific locations. These locations can be readily matched to specific fragments sizes (in kilobase pairs) by introduction into the separation technique of a marker, such as EcoR I digested λ bacteriophage DNA. In this manner, both the relative position of the bands to each other, as well as the absolute size of each band can be readily ascertained. The identifying genetic characterization for the unknown is then compared with characterizations present in a catalog or library. The catalog or library can consist of a book containing characterizations for at least two, and up to a vinually unlimited number of defined different organisms genera and species. For example, the number of pathologically relevant bacteria that cause human disease is estimated to be about 100, so it is estimated that a standard catalog of pathogenic bacteria would contain anywhere between 50 and 150 such characterizations.

The characterizations will depend on the type or types of endonuclease enzymes selected, possibly on the panicular organism used as the source for the radioactively labeled probe (the probe organism), and on the composition of the conserved DNA sequence information nucleic acids utilized to prepare the probe.

Thus, the catalog may, for each probe, contain a variety of enzyme-specific characterizations, with the size of each band listed, and with the relative intensity noted. As the concentration of the bound DNA bound to the filter decreases, only the most intense bands can be seen, and the size of this band or bands can thus identify species. Any variation or permutation of the above can of course be used for the library. Additionally, for a eukaryotic organism the library may contain patterns that result from the use of one type of DNA or any combination of organelle and/or nuclear DNA. The pattern for each DNA digest will depend on the probe composition. The catalog may be arranged so that if more than one strain or species is present in the extracted sample and detected by the probe, the resulting characterization can be interpreted.

A user can either compare the obtained characterization, e.g., band pattern, visually, or by aid of a one-dimensional, computer assisted, digital scanner programmed for recognition of patterns. These computer scanners are well known in the art of the time-of-sale transactions (the commonly utilized "supermarket" check-out bar code or pattern readers). Ideally, the library or catalog is present in a computer memory both in terms of the relative characterizations for a plurality of organisms, and in terms of the absolute values of molecular weight or size of the fragments. The catalog comparison then consists of matching the unknown characterization with one of the characterizations present in the library by means of either one or both of the stored information elements (relative characterizations and/or absolute size elements). The intensity of each band when compared to a standard can also reveal the amount of bound DNA hybridized, and thus can be used to estimate the extent of the presence of an organism, for example a prokaryote in a eukaryote.

If a user wishes to further confirm the nature and identification of a given organism, such user can digest the unknown with a second, different endonuclease, and compare the resulting characterization to catalog characterizations of the organism for the second chosen endonuclease. This process can be repeated as many times as necessary to get an accurate identification. Normally, however, a single analysis with a single probe would be sufficient in most instances.

The present invention and its variations can be used for a myriad of applications. It may be used by plant or animal breeders to correctly establish the species of their subjects, or it may be used by clinical and microbiological laboratories to establish the species of bacteria, parasites or fungi present in any medium, including in eukaryotic cells. In this latter use, the method is preferred to the standard microbiological assays, since it does not require microbiological assays, since it does not require isolation and growth of the microbes. *In vitro* growth and characterization is now either impossible for some microorganisms such as *Mycobacterium leprae* (agent of leprosy), impossible on standard media for some microorganisms such as the obligate intracellular bacteria (e.g. rickettsia, chlamydia, etc), or highly dangerous (e.g. *B anthracis* (agent of anthrax)). The present method depends on the isolation of nucleic acid and avoids these problems since it avoids conventional bacterial isolation and characterization. The method is expected to establish the species of microorganisms that have not yet been conventionally described. The method can be used by forensic laboratories to correctly and unambigously establish the species of plant or animal tissues in criminal investigations. It can also be used by entomologists to quickly establish insect species, when ascertaining the nature of crop infestations.

The method of this invention is preferably used for establishing the species of microorganisms wherever they may be found. These microorganisms may be found in physiological as well as non-physiological materials. They may be found in industrial growth media, culture broths, or the like, and may be concentrated for example by centrifugation. Preferably, the microorganisms are found in physiological media, most preferably they are found in animal sources infected therewith. In this latter embodiment, the method is used to diagnose bacterial infections in animals, most preferably in humans. The detection and identification of bacterial DNA with a prokaryotic probe is highly selective and occurs without hindrance, even in the presence of animal, (e.g., mammalian) DNA. If a prokaryotic probe is used, conditions can be selected which minimize hybridization with mitochondrial DNA, or mitochondrial bands can be subtracted from the pattern. The technique can thus be used in clinical laboratories, bacterial depositories, industrial fermentation laboratories, and the like.

When searching for infectious agents in specimens it is possible to search directly by extracting nucleic acid from the specimen, or by culturing first in media or cells to increase the number of agents, or by using a concentration step such as centrifugation, or by trying all approaches.

The present invention lends itself readily to the preparation of "kits" containing the elements necessary to carry out the process. Such a kit may comprise a carrier being compartmentalized to receive in close confinement therein one or more container means, such as tubes or vials. One of said container means may contain unlabeled or detectably labeled nucleic acid probe, such as for example the radioactively labeled cDNA to RNA from the organism probe, (preferably prokaryotic cDNA in the case of a kit to identify bacteria). The labeled nucleic acid probe may be present in lyophilized form, or in an appropriate buffer as necessary. One or more container means may contain one or more endonuclease enzymes to be utilized in digesting the DNA from the unknown organism. These enzymes may be present by themselves or in admixtures, in lyophilized form or in appropriate buffers. Ideally, the enzymes utilized in the kit are those for which corresponding catalogs have been prepared. Nothing stops the user, however, from preparing his or her own comparative standard at the moment of experiment. Thus, if a user suspects that an unknown is in fact of a given genus or species, he or she may prepare the identifying characteristics for the known and compare it with the characterization for the unknown. The kit may thus also contain all of the elements necessary in order to carry out this sub-process. These elements may include one or more known organisms, (such as bacteria), or isolated DNA from known organisms. In addition, the kit may also contain a "catalog", defined broadly as a booklet, or book, or pamphlet, or computer tape or disk, or computer access number, or the like, having the identifying characterizations for a variety of organisms of a certain group, such as plant species, mammal species, microbe species, especially pathologically important bacteria, insect species or the like. In this mode, a user would only need to prepare the characterization for the unknown organism, and then visually (or by computer) compare the obtained characterized with the characterizations in the catalog. The kit may also contain in one container probe RNA for probe synthesis, in another container radio-labeled deoxyribonucleoside triphosphate, and in another container primer. In this manner the user can prepare his or her own probe cDNA.

Finally, the kit may contain all of the additional elements necessary to carry out the technique of the invention, such as buffers, growth media, enzymes, pipettes, plates, nucleic acids, nucleoside triphosphates, filter paper, gel materials, transfer materials, autoradiography supplies, and the like. It may also contain antibiotic resistance sequence probes, viral probes, or other specific character probes.

Having now generally described this invention, the same will be better understood by reference to certain and specific examples which are included herein for purposes of illustration only and are not intended to be limiting of the invention, unless specified. The specific examples are preceded by a "materials and methods" section which, while relating to nucleic acid containing rRNA information and therefore not falling within the scope of the present invention, give a general indication to those skilled in the art of how certain steps relating to the invention may be carried out.

### Materials and Methods

### A. Bacterial

### Extraction of High Molecular Weight DNA

Bacterial broth cultures were centrifuged and the cells were washed with cold saline. The cells were suspended in a volume measured in ml of extraction buffer (0.15 M sodium chloride, 0.2 M EDTA, 0.03 M tris pH 8.5) approximately 10 times the gram weight of the packed cells. Lysozyme at 10 mg/ml was added to 0.5 mg/ml final concentration. The suspension was incubated at 37°C for 30 minutes. Cell disruption was completed by the addition of 25% SDS to 2.5% final concentration, and raising the temperature to 60°C for 10 minutes. After cooling in a tap water bath, mercaptoethanol was added to 1% final concentration. Pronase® at 20 mg/ml in 0.02 M tris pH 7.4 was predigested at 37°C for 2 hours and then added to 1 mg/ml final concentration. The solution was incubated at 37°C for 18 hours. Phenol was prepared by mixing one liter redistilled phenol, 2.5 liters double distilled water, 270 ml saturated Tris base, 12 ml mercaptoethanol, and EDTA to 10⁻³M final concentration and allowing the mixture to separate at 4°C. The phenol was washed with wash buffer (10⁻M sodium chloride, 10⁻³M EDTA, 10 mM tris pH 8.5). Then an equal volume of fresh buffer was added. Mercaptoethanol was added to 0.1% final concentration. The solution was mixed and stored at 4°C. One half volume prepared phenol and one half volume chloroform was added to the lysed cell solution. This was shaken for approximately 10 minutes and centrifuged at 3,400 × g for 15 minutes. The aqueous phase was removed with an inverted 25 ml glass pipette. The extraction procedure was repeated until there was little precipitate at the interface. One-ninth volume 2 N sodium acetate pH 5.5 was added to the aqueous phase. Two times volume of 95% ethyl alcohol at -20°C was poured slowly down the side of the flask. The end of a Pasteur pipette was melted close and used to spool the precipitated DNA. High molecular weight DNA was dissolved in buffer (10⁻³ EDTA, 10⁻²M tris pH 7.4). The concentration of DNA was determined by absorbance at 260 nm using 30 micrograms per absorbance unit as conversion factor.

### Restriction Endonuclease Digestion of DNA

EcoR I restriction endonuclease reactions were performed in 0.1 M tris-HCI pH 7.5, 0.05 M NaCl, 0.005 M MgCl₂, and 100 micrograms per ml bovine serum albumin. EcoR I reaction mixtures contained 5 units of enzyme per microgram of DNA, and were incubated four hours at 37°C. PST I restriction endonuclease reactions were performed in 0.006 M tris-HCI pH 7.4,0.05 M sodium chloride,0.006 M magnesium chloride, 0.006 M 2-mercaptoethanol, and 100 micrograms per ml of bovine serum albumin. PST I reaction mixtures contained 2 units of enzyme per microgram DNA, and were incubated four hours at 37°C. Usually 10 micrograms DNA was digested in a final volume of 40 microliters. Ten times concentration buffers were added. Steriie distilled water was added depending on the volume of DNA. A Bacteriophage DNA was restricted with EcoR I to provide marker bands for fragment size determinations. Usually 2 micrograms A DNA was digested with 20 units EcoR I in a final volume of 20 microliters.

### Gel Electrophoresis and DNA Transfer

DNA digests were supplemented with glycerol, to about 20%, and bromophenol blue tracking dye. In the case of A DNA digests, 20 microliters of 1x EcoR I buffer was added to each 20 microliter reaction mixture. Usually 15 microliters 75% glycerol and 5 microliters 0.5% bromophenol blue were added to each 40 microliter reaction mixture.

10 micrograms digested bacterial DNA or 2 micrograms digested λ DNA were loaded per well and overlaid with molten agarose. Digests were electrophoresed in 0.8% agarose with 0.02 M sodium acetate, 0.002 M EDTA, 0.018 M tris base, and 0.028 M tris HCI pH 8.05 at 35 V until the dye migrated 13 to 16 cm. Gels were then immersed in ethidium bromide (0.005 mg/ml) and placed on a UV-light box to visualize the λ fragments. DNA was transferred to nitrocellulose filter paper by the method of Southern, *supra.* Gels were treated with denaturing solution (1.5 M sodium chloride, 0.5 M sodium hydroxide) on a rocker table for 20 min. Denaturing solution was replaced with neutralization solution (3.0 M sodium chloride, 0.5 M tris HCl, pH 7.5), and after 40 minutes the gels were checked with pH paper. Following neutralization, the gels were treated with 6 x SSC buffer (SSC = 0.15 M sodium chloride, 0.015 M sodium citrate) for 10 minutes. DNA fragments were transferred from the gel to the nitrocellulose paper by drawing 6 × SSC through the gel and nitrocellulose paper with a stack of paper towels for 15 hours. Filters were placed between two sheets of 3 MM chromatography paper, wrapped in aluminum foil, shiny side out, and dried in a vacuum oven at 80°C for 4 hours.

### Synthesis of ³²P ribosomal RNA Complementary DNA (³²P rRNA cDNA)

³²P-labeled DNA complementary to *E. coli* R-13 23S and 16S ribosomal RNA was synthesized using reverse transcriptase from avian myeloblastosis virus (AMV). The reaction mixture contained 5 microliters 0.2 M dithiothreithol, 25 microliters 1 M tris pH 8.0, 8.3 microliters 3 M potassium chloride, 40 microliters 0.1 M magnesium chloride, 70 micrograms actinomycin, 14 microliters 0.04 M dATP, 14 microliters 0.04 M dGDP, 14 microliters 0.04 M dTTP and 96.7 microliters H₂O. The following were added to a plastic tube: 137.5 microliters reaction mixture, 15 microliters calf thymus primer (10 mg/ml), 7 microliters H 20, 3 microliters rRNA (using 40 micrograms/OD unit concentration, is 2.76 micrograms/microliters), 40 microliters deoxycitydine 5'-(³²P) triphosphate (10 mCi/ml), and 13 microliters AMV polymerase (6,900 units µl). The enzymatic reaction was incubated 1.5 hours at 37°C. Then the solution was extracted in 5 ml each of chloroform and prepared phenol. After centrifugation (JS 13,600 RPM), the aqueous phase was layered directly on a Sephadex® G-50 column (1.5 × 22 cm). A plastic 10 ml pipette was used for the column. A small glass bead was placed in the tip, rubber tubing with a pinch clamp was attached, and degassed G-50 swelled in 0.5% SDS overnight was added. The aqueous phase wall allowed to flow directly into the G-50 and was then eluted with 0.5% SDS. 20 fractions at 0.5 ml each were collected in plastic vials. Tubes containing peak fractions were detected by Cerenkov counting using a ³H discriminator, counting for 0.1 min. per sample and recording total counts. Peak fractions were pooled. Aliquots were added to Aquesol® (commercially available), and the CPM of ³²P per ml was determined by scintillation counting.

### Hybridization and Autoradiography

Fragments containing ribosomal RNA gene sequences were detected by autoradiography after hybridization of the DNA on the filters to ³²P-rRNA cDNA. Filters were soaked in hybridization mix (3 × SSC, 0.1% SDS, 100 micrograms/ml denatured and sonicated canine DNA, and Deinhart's solution (0.2% each of bovine serum albumen, Ficoll, and polyvinyl pyrrolidine)), for 1 hour at 68°C. ³²P rRNA cDNA was added at 4 × 10⁶ CPM/ml, and the hybridization reaction was incubated at 68°C for 48 hours. Filters were then washed in 3 × SSC, and 0.1 % SDS at 15 min. intervals for 2 hours or until the wash solution contained about 3,000 cpm ³²P per ml. Filters were air dried, wrapped in plastic wrap and autoradiographed approximately 1 hour with Kodak X-OMAT R film at -70°C.

B. Mammalian experiments. *Mus musculus domesticus* (mouse) rRNA probes were synthesized from 18S and 28S, and only 28S rRNA. Nucleic acid was extracted from mouse liver and precipitated. High molecular weight DNA was spooled and removed. The remaining nucleic acid was collected by centrifugation and dissolved in buffer, 50 mM MgCl₂ and 100 mM Tris pH 7.4. DNAse (RNAse free) was added to a concentration of 50 µg/m. The mixture was incubated at 37°C for 30 min. The resulting RNA was re-extracted, ethanol precipitated, and dissolved in 1 mM sodium phosphate buffer pH 6.8 A 5 to 20% sucrose gradient in 0.1M Tris pH 7.4 and 0.1M EDTA was prepared. The sample was added and the gradients spun in an SW40 rotor 7 hr. at 35K RPM. Fractions were collected by optical density. The 18S and 28S fractions were selected by comparison to known molecular weight markers.

For all of the mammalian experiments relaxed hybridization conditions were used, 54°C. The washing procedure, carried out at 54°C, was 3 separate washes with 3×SSC with 0.05% SDS for 15 min. each.

Examples 1 to 3 describe computer simulations utilizing a histone gene information-containing probe, a tryptophan operon trp D gene-information containing probe, and an α-fetoprotein gene information-containing probe, respectively.

### Example 1

### Use of an H4 Histone Gene Probe

A computer simulation of the identification and characterization of two animal species (sea urchin and mouse) was carried out using a conserved DNA sequence derived from the H4 histone gene.

The histone H4 gene sequence for sea urchin (Psammechinus miliaris) is shown below, where A, T, C, G, represent the known nucleotides, and N represent a presently unknown position (788 base pairs). 
        10         20         30         40         50
CAACATATTA GAGGAAGGGA GAGAGAGAGA GAGAGAGAGA GAGAGAGAGA
GTTGTATAAT CTCCTTCCCT CTCTCTCTCT CTCTCTCTCT CTCTCTCTCT

        60         70         80         90        100
GGGGGGGGGG GAGGGAGAAT TGCCCAAAAC ACTGTAAATG TAGCGTTAAT
CCCCCCCCCC CTCCCTCTTA ACGGGTTTTG TGACATTTAC ATCGCAATTA

       110        120        130        140        150
GAACTTTTCA TCTCATCGAC TGCGCGTGTA TAAGGATGAT TATAAGCTTT
CTTGAAAAGT AGAGTAGCTG ACGCGCACAT ATTCCTACTA ATATTCGAAA

       160        170        180        190        200
TTTTCAATTT ACAGGCACTA CGTTACATTC AAATCCAATC AATCATTTGA
AAAAGTTAAA TGTCCGTGAT GCAATGTAAG TTTAGGTTAG TTAGTAAACT

       210        220        230        240        250
ATCACCGTCG CAAAAGGCAG ATGTAAACTG TCAAGTTGTC AGATTGTGTG
TAGTGGCAGC GTTTTCCGTC TACATTTGAC AGTTCAACAG TCTAACACAC

       260        270        280        290        300
CGCGGCCTCC AGTGAGCTAC CCACCGGGCC GTCGCGGAGG GGCGCACCTG
GCGCCGGAGG TCACTCGATG GGTGGCCCGG CAGCGCCTCC CCGCGTGGAC
 
310 320 330 340 350
TGCGGGAGGG GTCATCGGAG GGCGATCGAG CCTCGTCATC CAAGTCCGCA
ACGCCCTCCC CAGTAGCCTC CCGCTAGCTC GGAGCAGTAG GTTCAGGCGT

360 370 380 390 400
TACGGGTGAC AATACCCCCG CTCACCGGGA GGGTTGGTCA ATCGCTCAGC
ATGCCCACTG TTATGGGGGC GAGTGGCCCT CCCAACCAGT TAGCGAGTCG

410 420 430 440 450
GAAACGTCCA GTCGTCAGCA TCGCACTAAG ACTCTCTCTC AATCTCCATA
CTTTGCAGGT CAGCAGTCGT AGCGTGATTC TGAGAGAGAG TTAGAGGTAT

460 470 480 490 500
ATGTCAGGCC GTGGTAAAGG AGGCAAGGGG CTCGGAAAGG GAGGCGCCAA
TACAGTCCGG CACCATTTCC TCCGTTCCCC GAGCCTTTCC CTCCGCGGTT

510 520 530 540 550
GCGTCATCGC AAGGTCCTAC GAGACAACAT CCAGGGCATC ACCAAGCCTG
CGCAGTAGCG TTCCAGGATG CTCTGTTGTA GGTCCCGTAG TGGTTCGGAC

560 570 580 590 600
CAATCCGCCG ACTCNNNNNN NNNNNNNNNN NNNNNNGAAT CTCTGGTCTT
GTTAGGCGGC TGAGNNNNNN NNNNNNNNNN NNNNNNCTTA GAGACCAGAA

610 620 630 640 650
ATCTACGAGG AGACACGAGG GGTGCTGAAG GNNNNNNNNN NNNNNNNNNN
TAGATGCTCC TCTGTGCTCC CCACGACTTC CNNNNNNNNN NNNNNNNNNN

660 670 680 690 700
NNNNNNNNNN NNNNNNNNNN NNNNNNNNNN NNNNNNNNNN NNNNNNNNNN
NNNNNNNNNN NNNNNNNNNN NNNNNNNNNN NNNNNNNNNN NNNNNNNNNN

710 720 730 740 750
NNNNNNNNNN NNNNNNNNNN NNNNNNNNNN NNGGCCGAAC ACTGTACGGC
NNNNNNNNNN NNNNNNNNNN NNNNNNNNNN NNCCGGCTTG TGACATGCCG

760 770 780
TTCGGCGGCT AAGTGAAGCA GACTTGGCTA GAATAACG
AAGCCGCCGA TTCACTTCGT CTGAACCGAT CTTATTGC
 The analogous mouse H4 gene sequence is shown below (968 base pairs):

10 20 30 40 50
GAATTCTCCG AGGGACTTCG GCACCATAAT TAAGAAAATC GAAAATAAAA
CTTAAGAGGC TCCCTGAAGC CGTGGTATTA ATTCTTTTAG CTTTTATTTT

60 · 70 80 90 100
AAATAAAGGC TTGAGACTGT AAGGAACCGG TAGAGGGCAG AGAAGAGAAA
TTTATTTCCG AACTCTGACA TTCCTTGGCC ATCTCCCGTC TCTTCTCTTT

110· 120 130 140 150
AGAAAAACAG GAAGATGATG CAACATCCAG AGCCCGGATA ATTTAGAAAG
TCTTTTTGTC CTTCTACTAC GTTGTAGGTC TCGGGCCTAT TAAATCTTTC

160 170 180 190 200
GTTCCCGCCC GCGCGCTTTC AGTTTTCAAT CTGGTCCGAT CCTCTCATAT
CAAGGGCGGG CGCGCGAAAG TCAAAAGTTA GACCAGGCTA GGAGAGTATA

210 220 230 240 250
ATTAGTGGCA CTCCACCTCC AATGCCTCAC CAGCTGGTGT TTCAGATTAC
TAATCACCGT GAGGTGGAGG TTACGGAGTG GTCGACCACA AAGTCTAATG

260 270 280 290 300
ATTAGCTATG TCTGGCAGAG GAAAGGGTGG AAAGGGTCTA GGCAAGGGTG
TAATCGATAC AGACCGTCTC CTTTCCCACC TTTCCCAGAT CCGTTCCCAC

310 320 330 340 350
GCGCCAAGCG CCATCGCAAA GTCTTGCGTG ACAACATCCA GGGTATCACC
CGCGGTTCGC GGTAGCGTTT CAGAACGCAC TGTTGTAGGT CCCATAGTGG

360 370 380 390 400
AAGCCCGCCA TCCGCCGCCT GGCTCGGCGC GGTGGGGTCA AGCGCATCTC
TTCGGGCGGT AGGCGGCGGA CCGAGCCGCG CCACCCCAGT TCGCGTAGAG

410 420 430 440 450
CGGCCTCATC TACGAGGAGA CCCGTGGTGT GCTGAAGGTG TTCCTGGAGA
GCCGGAGTAG ATGCTCCTCT GGGCACCACA CGACTTCCAC AAGGACCTCT

460 470 480 490 500
ACGTCATCCG CGACGCAGTC ACCTACACCG AGCACGCCAA GCGCAAGACC
TGCAGTAGGC GCTGCGTCAG TGGATGTGGC TCGTGCGGTT CGCGTTCTGG

510 520 530 540 550
GTCACCGCTA TGGATGTGGT GTACGCTCTC AAGCGCCAGG GCCGCACCCT
CAGTGGCGAT ACCTACACCA CATGCGAGAG TTCGCGGTCC CGGCGTGGGA

560 570 580 590 600
CTACGGCTTC GGAGGCTAGA CGCCGCCGCT TCAATTCCCC CCCCCCCCCC
GATGCCGAAG CCTCCGATCT GCGGCGGCGA AGTTAAGGGG GGGGGGGGGG

610 620 630 640 650
ATCCCTAACG GCCCTTTTTA GGGCCAACCA CAGTCTCTTC AGGAGAGCTG
TAGGGATTGC CGGGAAAAAT CCCGGTTGGT GTCAGAGAAG TCCTCTCGAC

660 670 680 690 700
ACACTGACTT GGGTCGTACA GGTAATAACC GCGGGTTTAG GACTCACGCT
TGTGACTGAA CCCAGCATGT CCATTATTGG CGCCCAAATC CTGAGTGCGA

710 720 730 740 750
ACTAGGTGTT CCGCTTTTAG AGCCATCCAC TTAAGTTTCT ATACCACGGC
TGATCCACAA GGCGAAAATC TCGGTAGGTG AATTCAAAGA TATGGTGCCG

760 770 780 790 800
GGATAGATAG CATCCAGCAG GGTCTGCTCA CACTGGGAAT TTTAATTCCT
CCTATCTATC GTAGGTCGTC CCAGACGAGT GTGACCCTTA AAATTAAGGA

810 820 830 840 850
ACTTAGGGTG TGAGCTGGTT GTCAGGTCAA GAGACTGGCT AAGATTTTCT
TGAATCCCAC ACTCGACCAA CAGTCCAGTT CTCTGACCGA TTCTAAAAGA

860 870 880 890 900
TTAGCTCGTT TGGAGCAGAA TTGCAATAAG GAGACCCTTT GGATGGGATG
AATCGAGCAA ACCTCGTCTT AACGTTATTC CTCTGGGAAA CCTACCCTAC

910 920 930 940 950
ACCTATGTCC ACACATCAAA TGGCTATGTG GCTGTGTCCC TGTGTTTCCA
TGGATACAGG TGTGTAGTTT ACCGATACAC CGACACAGGG ACACAAAGGT

960
ATGAGTGGCT GTGCTTGA
TACTCACCGA CACGAACT

The region of homology for both aforementioned sequences is shown below, where asterisks denote not homologous portions. Within the region shown, the first 118 base pairs have 80.5% homology and are used as a conserved DNA sequence probe in this example (sea urchin (top) base positions 449 to 567, mouse (bottom) base positions 257 to 375):

 . *    *    * *  *       **  *   *    *   *   *   *    *              *
449  TAATGTCAGGCCGTGGTAAAGG-AGGCAA-GGGGCTCGG-AAAGGGAGGCGCCAAGCGTC
257  TA-TGTCTGGCAGAGG-AAAGGGTGG-AAAGGGTCTAGGCAA-GGGTGGCGCCAAGCGCC

      *   * *  *              *           *  *         *  **
ATCGCAAGGTCCTACGAGACAACATCCAGGGCATCACCAAGCCTGCAATCCGCCGACT-C
ATCGCAAAGTCTTGCGTGACAACATCCAGGGTATCACCAAGCCCGCCATCCGCCGCCTGG

                    **     *  * * *                 *  **  *
NNNNNNNNNNNNNNNNNNNNNNGAATCTCTGGTCTT-ATCTACGAGGAGACACGAGGG-G
CTCGGCGCGGTGGGGTCAAGCG-CATCTCCGG-CCTCATCTACGAGGAGACCCG-TGGTG

TGCTGAAGGNNNNNNNNNNNNNNNNNNNNNNNNNNNNNNNNNNNNNNNNNNNNNNNNNNN
TGCTGAAGGTGTTCCTGGAGAACGTCATCCGCGACGCAGTCACCTACACCGAGCACGCCA

                                                    * * *
NNNNNNNNNNNNNNNNNNNNNNNNNNNNNNNNNNNNNNNNNNNNNNNNNNGGCCG-AAC-
AGCGCAAGACCGTCACCGCTATGGATGTGGTGTACGCTCTCAAGCGCCAGGGCCGCACCC

*  **          *      ***  *   * * **  ** *
ACTGTACGGCTTCGGCGGCTAAGTGAAGCAGAC--TTGGCTA    780
TCT--ACGGCTTCGGAGGCTA---GACGCCGCCGCTT--CAA    584

% = 84.503
P( 342, 289) = .000E+00     E = .000

Restriction endonuclease cleavage sites were determined from the two sequences. A list of cleavage sites for the sea urchin and mouse sequences is shown below. Numbers indicate the 5' side of the cleavage site, unless site name is in brackets, which indicates that only the recognition site is known.

| SEA URCHIN | | | | | | | |
|---|---|---|---|---|---|---|---|
| Sequence | Appears at position | | | | | | |
| Acyl (GPCGQC) | 495 | | | | | | |
| Alul (AGCT) | 147 | 267 | | | | | |
| Asul (GGNCC) | 277 | 514 | | | | | |
| Avall (GGLCC) | 514 | | | | | | |
| Caull (CCMGG) | 276 | 377 | | | | | |
| Ddel (CTNAG) | 396 | 427 | | | | | |
| DpnI (GATC) | 326 | | | | | | |
| EcoRl* (PPATQQ) | 184 | | | | | | |
| EcoRII (CCLGG) | 531 | | | | | | |
| Fnu4Hl (GCNGC) | 254 | | | | | | |
| FnuDII (CGCG) | 125 | 253 | 285 | | | | |
| Fokl (GGATG) | 148 | | | | | | |
| Fokl (CATCC) | 324 | 515 | | | | | |
| Haell (PGCGCQ) | 498 | | | | | | |
| HaeIII (GGCC) | 256 | 279 | 459 | | | | |
| Hsal (GCGTC) | 491 | | | | | | |
| HsiCI (GGQPCC) | 494 | | | | | | |
| HsiJII (GPGCQC) | 483 | | | | | | |
| Hhal (GCGC) | 125 | 253 | 295 | 497 | | | |
| HindIII (AAGCTT) | 145 | | | | | | |
| Hinfl (GANTC) | 200 | 431 | 561 | | | | |
| Hpall (GCGG) | 275 | 376 | | | | | |
| Hphl (GGTGA) | 368 | | | | | | |
| Hphl (TCACC) | 195 | 365 | 532 | | | | |
| Mbol (GATC) | 324 | | | | | | |
| MnII (CCTC) | 267 | 342 | | | | | |
| MnII (GAGG) | 4 | 42 | 54 | 280 | 299 | 311 | |
| | 372 | 463 | 484 | | | | |
| Narl (GGCGCC) | 495 | | | | | | |
| NspII (GCMGC) | 256 | | | | | | |
| Pvul (CGATCG) | 327 | | | | | | |
| ScrFI (CCNGG) | 276 | 377 | 533 | | | | |
| SfaNI (GCATC) | 409 | 527 | | | | | |
| Taql (TCGA) | 117 | 327 | | | | | |

| MOUSE | | | | | | | |
|---|---|---|---|---|---|---|---|
| Sequence | Appears at position | | | | | | |
| Acyl (GPCGQC) | 302 | 571 | | | | | |
| AfIII (CTTAAG) | 731 | | | | | | |
| Alul (AGCT) | 234 | 256 | 648 | 815 | 855 | | |
| Asul (GGNCC) | 184 | 540 | 611 | 622 | | | |
| Avall (GGLCC) | 184 | | | | | | |
| BssHII (GCGCGC) | 162 | | | | | | |
| Caull (CCMGG) | 135 | | | | | | |
| Ddel (CTNAG) | 803 | 840 | | | | | |
| DpnI (GATC) | 190 | | | | | | |
| [EcoB] (AGCANNNNNNNNTCA) | 766 | | | | | | |
| [EcoP1] (AGACC) | 418 | 496 | 882 | | | | |
| [EcoP1] (GGTCT) | 285 | 771 | | | | | |
| [EcoP15] (CAGCAG) | 765 | | | | | | |
| EcoRI (GAATTC) | 2 | | | | | | |
| EcoRI* (PPATQQ) | 4 | 790 | 845 | | | | |
| EcoRII (CCLGG) | 338 | 368 | 443 | 536 | | | |
| Fnu4HI (GCNGC) | 366 | 543 | 574 | 577 | | | |
| FnuDII (CGCG) | 162 | 164 | 380 | 461 | 682 | | |
| Fokl (GGATG) | 526 | 905 | 910 | | | | |
| Fokl (CATCC) | 111 | 322 | 346 | 442 | 587 | 711 | 748 |
| Haell (PGCGCQ) | 305 | 312 | 537 | | | | |
| HaeIII (GGCC) | 404 | 542 | 612 | 624 | | | |
| HsaI (GACGC) | 472 | 579 | | | | | |
| HsiAI (GLGCLC) | 485 | | | | | | |
| HsiCI (GGQPCC) | 21 | 301 | | | | | |
| HsiJII (GPGCQC) | 135 | | | | | | |
| Hhal (GCGC) | 164 | 166 | 304 | 311 | 380 | 395 | |
| | 494 | 536 | | | | | |
| Hinfl (GANTC) | 692 | | | | | | |
| Hpall (CCGG) | 78 | 135 | 401 | | | | |
| Hphl (TCACC) | 220 | 339 | 462 | 495 | | | |
| MboI (GATC) | 188 | | | | | | |
| MboII (GAAGA) | 105 | 124 | | | | | |
| MboII (TCTTC) | 629 | | | | | | |
| MnII (CCTC) | 202 | 227 | 236 | 415 | 559 | | |
| MnII (GAGG) | 3 | 76 | 261 | 407 | 555 | | |
| NarI (GGCGCC) | 302 | | | | | | |
| NspII (GCMGC) | 368 | 545 | 576 | 579 | | | |
| PvuII (GAGCTG) | 234 | | | | | | |
| RsaI (GTAC) | 523 | 668 | | | | | |
| SacII (CCGCGG) | 683 | | | | | | |
| ScrFI (CCNGG) | 135 | 340 | 370 | 445 | 538 | | |
| SfaNI (GATGC) | 127 | | | | | | |
| SfaNI (GCATC) | 385 | 751 | | | | | |
| TaqI (TCGA) | 40 | | | | | | |
| Th111I (GACNNNGTC) | 466 | | | | | | |
| Tth111II (TGQTTG) | 953 | | | | | | |
| XmnI (GAANNNNTTC) | 439 | | | | | | |

The sea urchin and mouse sequences are compared with Hha I (GCGC) and the described probe sequence. The sea urchin sequence has cleavage sites at positions 295 and 497, thus creating a 202 bp fragment, which, if denatured, would hybridize with the probe sequence. Hha I IGCGC) sites in the mouse sequence, 166, 304, 311 and 380, indicate that fragments of *69* and *138* could be detected with the probe sequence.

Thus the genetic characterization for sea urchin is
202
while that for mouse is
69
138

### Example 2

### Use of trp D Gene of the Tryptophan Operon as a Probe

The same type of computer simulation as in Example 1 was carried out using trp D gene as a probe. This allows the conclusion that *E. coli* and *Salmonella typhimurium* can be distinguished by restriction fragments containing a conserved sequence.

The *E. coli* trp D gene with 684 bp's is shown below: 
10 20 30 40 50
GAAGCCGACG AAACCCGTAA CAAAGCTCGC GCCGTACTGC GCGCTATTGC
CTTCGGCTGC TTTGGGCATT GTTTCGAGCG CGGCATGACG CGCGATAACG

60 70 80 90 100
CACCGCGCAT CATGCACAGG AGACTTTCTG ATGGCTGACA TTCTGCTGCT
GTGGCGCGTA GTACGTGTCC TCTGAAAGAC TACCGACTGT AAGACGACGA

110 120 130 140 150
CGATAATATC GACTCTTTTA CGTACAACCT GGCAGATCAG TTGCGCAGCA
GCTATTATAG CTGAGAAAAT GCATGTTGGA CCGTCTAGTC AACGCGTCGT

160 170 180 190 200
ATGGGCATAA CGTGGTGATT TACCGCAACC ATATACCGGC GCAAACCTTA
TACCCGTATT GCACCACTAA ATGGCGTTGG TATATGGCCG CGTTTGGAAT

210 220 230 240 250
ATTGAACGCT TGGCGACCAT GAGTAATCCG GTGCTGATGC TTTCTCCTGG
TAACTTGCGA ACCGCTGGTA CTCATTAGGC CACGACTACG AAAGAGGACC

260 270 280 290 300
CCCCGGTGTG CCGAGCGAAG CCGGTTGTAT GCCGGAACTC CTCACCCGCT
GGGGCCACAC GGCTCGCTTC GGCCAACATA CGGCCTTGAG GAGTGGGCGA

310 320 330 340 350
TGCGTGGCAA GCTGCCCATT ATTGGCATTT GCCTCGGACA TCAGGCGATT
ACGCACCGTT CGACGGGTAA TAACCGTAAA CGGAGCCTGT AGTCCGCTAA

360 370 380 390 400
GTCGAAGCTT ACGGGGGCTA TGTCGGTCAG GCGGGCGAAA TTCTCCACGG
CAGCTTCGAA TGCCCCCGAT ACAGCCAGTC CGCCCGCTTT AAGAGGTGCC

410 420 430 440 450
TAAAGCCTCC AGCATTGAAC ATGACGGTCA GGCGATGTTT GCCGGATTAA
ATTTCGGAGG TCGTAACTTG TACTGCCAGT CCGCTACAAA CGGCCTAATT

460 470 480 490 500
CAAACCCGCT GCCGGTGGCG CGTTATCACT CGCTGGTTGG CAGTAACATT
GTTTGGGCGA CGGCCACCGC GCAATAGTGA GCGACCAACC GTCATTGTAA

510 520 530 540 550
CCGGCCGGTT TAACCATCAA CGCCCATTTT AATGGCATGG TGATGGCAGT
GGCCGGCCAA ATTGGTAGTT GCGGGTAAAA TTACCGTACC ACTACCGTCA

560 570 580 590 600
ACGTCACGAT GCGGATCGCG TTTGTGGATT CCAGTTCCAT CCGGAATCCA
TGCAGTGCTA CGCCTAGCGC AAACACCTAA GGTCAAGGTA GGCCTTAGGT

610 620 630 640 650
TTCTCACCAC CCAGGGCGCT CGCCTGCTGG AACAAACGCT GGCCTGGGCG
AAGAGTGGTG GGTCCCGCGA GCGGACGACC TTGTTTGCGA CCGGACCCGC

660 670 680
CAGCATAAAC TAGAGCCAGC CAACACGCTG CAA
GTCGTATTTG ATCTCGGTCG GTTGTGCGAC GTT
 The trp D gene, 683 base pairs, for *S. typhimurium* is shown below:

10 20 30 40 50
GAAGCCGATG AAACCCGTAA TAAAGCGCGC GCCGTATTGC GTGCTATCGC
CTTCGGCTAC TTTGGGCATT ATTTCGCGCG CGGCATAACG CACGATAGCG

60 70 80 90 100
CACCGCGCAT CATGCACAGG AGACCTTCTG ATGGCTGATA TTCTGCTGCT
GTGGCGCGTA GTACGTGTCC TCTGGAAGAC TACCGACTAT AAGACGACGA

110 120 130 140 150
CGATAACATC GACTCGTTCA CTTGGAACCT GGCAGATCAG CTACGAACCA
GCTATTGTAG CTGAGCAAGT GAACCTTGGA CCGTCTAGTC GATGCTTGGT

160 170 180 190 200
ACGGTCATAA CGTGGTGATT TACCGTAACC ATATTCCGGC GCAGACGCTT
TGCCAGTATT GCACCACTAA ATGGCATTGG TATAAGGCCG CGTCTGCGAA

210 220 230 240 250
ATCGATCGCC TGGCGACAAT GAAAAATCCT GTGCTAATGC TCTCCCCCGG
TAGCTAGCGG ACCGCTGTTA CTTTTTAGGA CACGATTACG AGAGGGGGCC

260 270 280 290 300
CCCGGGTGTT CCCAGCGAGG CAGGTTGTAT GCCGGAGCTG CTGACCCGAC
GGGCCCACAA GGGTCGCTCC GTCCAACATA CGGCCTCGAC GACTGGGCTG

310 320 330 340 350
TACGCGGCAA GTTACCGATC ATCGGCATTT GTCTGGGGCA TCAGGCGATT
ATGCGCCGTT CAATGGCTAG TAGCCGTAAA CAGACCCCGT AGTCCGCTAA

360 370 380 390 400
GTCGAAGCTT ACGGCGGTTA CGTCGGTCAG GCGGGAGAAA TCCTGCATGG
CAGCTTCGAA TGCCGCCAAT GCAGCCAGTC CGCCCTCTTT AGGACGTACC

410 420 430 440 450
CAAAGCCTCC AGCATTGAGC ATGACGGTCA GGCGATGTTC GCCGGGCTCG
GTTTCGGAGG TCGTAACTCG TACTGCCAGT CCGCTACAAG CGGCCCGAGC

460 470 480 490 500
CGAATCCGCT ACCGGTCGCG CGTTATCATT CGCTGGTCGG CAGTAATGTT
GCTTAGGCGA TGGCCAGCGC GCAATAGTAA GCGACCAGCC GTCATTACAA

510 520 530 540 550
CCTGCCGGGC TGACCATTAA CGCCCATTTC AACGGCATGG TGATGGCGGT
GGACGGCCCG ACTGGTAATT GCGGGTAAAG TTGCCGTACC ACTACCGCCA

560 570 580 590 600
ACGTCATGAT GCGGATCGCG TTTGCGGTTT TCAATTTCAT CCCGAGTCCA
TGCAGTACTA CGCCTAGCGC AAACGCCAAA AGTTAAAGTA GGGCTCAGGT

610 620 630 640 650
TCCTGACGAC ACAGGGCGCG CGTCTACTGG AGCAAACATT AGCCTGGGCG
AGGACTGCTG TGTCCCGCGC GCAGATGACC TCGTTTGTAA TCGGACCCGC

660 670 680
CTGGCGAAGC TGGAACCGAC CAACACCCTA CAG
GACCGCTTCG ACCTTGGCTG GTTGTGGGAT GTC

Two homology regions between both sequences were next established, where the upper sequence is for *E. coli* and the lower one for *S. typhimurium:* 
Region I

452      **     *     *           *        *        **    * * *  **
453    AAACCCGCTGCCGGTGGCGCGTTATCACTCGCTGGTTGGCAGTAACATTCC-GGCCGG-T
       AA-TCCGCTACCGGTCGCGCGTTATCATTCGCTGGTCGGCAGTAATGTTCCTGCC-GGGC

        * *    *           *  *              *        *
       TTAACCATCAACGCCCATTTTAATGGCATGGTGATGGCAGTACGTCACGATGCGGATCGC
       TGA-CCATTAACGCCCATTTCAACGGCATGGTGATGGCGGTACGTCATGATGCGGATCGC

            *  **   * *  *     *  *     *  *  *  *        *  *  *
       GTTTGTGG-ATTCCAGTTCCATCCGGAATCCATTCTCACCACCCAGGGCGCTCGGCTGCT
       GTTTGCGGTTTTC-AATTTCATCCCGAGTCCATCCTGACGACACAGGGCGCGCGTCTACT

          *     ** *          **  **  *  *  *  * * *     *  *
       GGAACAAACGCTGGCCTGGGCGC-AGCATAAACTAGAGCC-AGCCAACACGCTGCA    682
       GGAGCAAACATTAGCCTGGGCGCTGGC-GAAGCTGGAACCGACC-AACACCCTACA    662

       % = 78.390
       P( 236, 185) = .000E+00      E = .000
 
Region II

*          **  **  *   **    *    *     *
1    GAAGCCGACGAAACCCGTAA-CAA-AGCTCGCGCCGTACTGCGCGCTATTGCCACCGCGC
1    GAAGCCGATGAAACCCGTAATAAAGCGCGCGC--CGTATTGCGTGCTATCGCCACCGCGC

*  *          *                *      *
ATCATGCACAGGAGAC-TTTCTGATGGCTGACATTCTGCTGCTCGATAATATCGACTC-T
ATCATGCACAGGAGACCTT-CTGATGGCTGATATTCTGCTGCTCGATAACATCGACTCGT

**  ** * * * *           * *  * * *  **  *
TTTAC--GTACAACCTGGCAGATCAGTTGCGCAGC-AATGGG-CATAACGTGGTGATTTA
T-CACTTGGA-ACC-TGGCAGATCAGCTACG-AACCAA-CGGTCATAACGTGGTGATTTA

*        *        *  *    * *  *   * * *    *    **  *
CCGCAACCATATACCGGCGCAAAC-CTTAATTGAACGC-TTGGCGACCATGAGTAA-TCC
CCGTAACCATATTCCGGCGCAGACGCTTA-TCGATCGCCTGG-CGACAATGA-AAAATCC

*     *     * * *  *     *  *   **  * * ** *  *
GGTGCTGATGCT-TTCTCCTGGCCCCGG-TGT-GCC-GAGCGAAGCCGGTTGTATGCCGG
TGTGCTAATGCTCTCC-CCCGGCCC-GGGTGTTCCCAGCG--AGGCAGGTTGTATGCCGG

*  *  *     *  **   **     * *  *  *  *        *  **  *
AACTCCTCACCCG-CT-TGCGTGGCAAGCTGCCCATTATTGGCATTTGCCT-CGGACATC
AGCTGCTGACCCGACTACGCG--GCAAGTTACCGATCATCGGCATTTGTCTGGGG-CATC

*  **  *             *     *  *  *  *
AGGCGATTGTCGAAGCTTACGG-GGGCTATGTCGGTCAGGCGGGCGAAATTCTCCACGG-
AGGCGATTGTCGAAGCTTACGGCGG-TTACGTCGGTCAGGCGGGAGAAATCCTGCATGGC

*  *              *                    *
TAAAGCCTCCAGCATTGAACATGACGGTCAGGCGATGTTTGCCGG     445
AAA-GCCTCCAGCATTGAGCATGACGGTCAGGCGATGTTCGCCGG     445

% = 80.215
P( 465, 373) = .000E+00     E = .000
 Restriction sites in both sequences are shown as follows:

| | E. coli | | | | | | | |
|---|---|---|---|---|---|---|---|---|
| Hpall (CCGG) | 187 | 229 | 254 | 272 | 283 | 443 | 463 | 502 |
| | 506 | 592 | | | | | | |
| Hphl (GGTGA) | 177 | 552 | | | | | | |
| Hphl (TCACC) | 285 | 597 | | | | | | |
| Mbol (GATC) | 135 | 564 | | | | | | |

| | S. typhimurium | | | | | | | |
|---|---|---|---|---|---|---|---|---|
| Hpall (CCGG) | 187 | 248 | 253 | 283 | 443 | 463 | 506 | |
| Hphl (GGTGA) | 177 | 552 | | | | | | |
| Mbol (GATC) | 135 | 204 | 317 | 564 | | | | |
| MnII (CCTC) | 417 | | | | | | | |

The *E. coli* sequence has Mbo I (GATC) sites at 135 and 564. There is a 429 bp fragment that can be detected by both Region 1 and 2 probes. The same enzyme has sites at 135, 204, 317, and 564 in the *S. typhimurium* sequence. A probe of the two homology regions would detect fragments of *69*, *113* and *247* bp.

Thus the identifying genetic characterization for *E. coli* with this probe and enzyme is
429
whereas that for *S. typhimurium* is
69
113
247

### Example 3

### Use of α-Fetoprotein Gene as a Probe

This example shows the use of a region of homology in the α-fetoprotein gene sequence of human and rat, and the endonuclease MnII (GAGG).

The human α-fetoprotein message cDNA (1578 bp's) is as follows: 
10 20 30 40 50
AGCTTGGCAT AGCTACCATC ACCTTTACCC AGTTTGTTCC GGAAGCCACC
TCGAACCGTA TCGATGGTAG TGGAAATGGG TCAAACAAGG CCTTCGGTGG

60 70 80 90 100
GAGGAGGAAG TGAACAAAAT GACTAGCGAT GTGTTGGCTG CAATGAAGAA
CTCCTCCTTC ACTTGTTTTA CTGATCGCTA CACAACCGAC GTTACTTCTT

110 120 130 140 150
AAACTCTGGC GATGGGTGTT TAGAAAGCCA GCTATCTGTG TTTCTGGATG
TTTGAGACCG CTACCCACAA ATCTTTCGGT CGATAGACAC AAAGACCTAC

160 170 180 190 200
AAATTTGCCA TGAGACGGAA CTCTCTAACA AGTATGGACT CTCAGGCTGC
TTTAAACGGT ACTCTGCCTT GAGAGATTGT TCATACCTGA GAGTCCGACG

210 220 230 240 250
TGCAGCCAAA GTGGAGTGGA AAGACATCAG TGTCTGCTGG CACGCAAGAA
ACGTCGGTTT CACCTCACCT TTCTGTAGTC ACAGACGACC GTGCGTTCTT
 
260 270 280 290 300
GACTGCTCCG GCCTCTGTCC CACCCTTCCA GTTTCCAGAA CCTGCCGAGA
CTGACGAGGC CGGAGACAGG GTGGGAAGGT CAAAGGTCTT GGACGGCTCT

310 320 330 340 350
GTTGCAAAGC ACATGAAGAA AACAGGGCAG TGTTCATGAA CAGGTTCATC
CAACGTTTCG TGTACTTCTT TTGTCCCGTC ACAAGTACTT GTCCAAGTAG

360 370 380 390 400
TATGAAGTGT CAAGGAGGAA CCCCTTCATG TATGCCCCAG CCATTCTGTC
ATACTTCACA GTTCCTCCTT GGGGAAGTAC ATACGGGGTC GGTAAGACAG

410 420 430 440 450
CTTGGCTGCT CAGTACGACA AGGTCGTTCT GGCATGCTGC AAAGCTGACA
GAACCGACGA GTCATGCTGT TCCAGCAAGA CCGTACGACG TTTCGACTGT

460 470 480 490 500
ACAAGGAGGA GTGCTTCCAG ACAAAGAGAG CATCCATTGC AAAGGAATTA
TGTTCCTCCT CACGAAGGTC TGTTTCTCTC GTAGGTAACG TTTCCTTAAT

510 520 530 540 550
AGAGAAGGAA GCATGTTAAA TGAGCATGTA TGTTCAGTGA TAAGAAAATT
TCTCTTCCTT CGTACAATTT ACTCGTACAT ACAAGTCACT ATTCTTTTAA

560 570 580 590 600
TGGATCCCGA AACCTCCAGG CAACAACCAT TATTAAGCTA AGTCAAAAGT
ACCTAGGGCT TTGGAGGTCC GTTGTTGGTA ATAATTCGAT TCAGTTTTCA

610 620 630 640 650
TAACTGAAGC AAATTTTACT GAGATTCAGA AGCTGGCCCT GGATGTGGCT
ATTGACTTCG TTTAAAATGA CTCTAAGTCT TCGACCGGGA CCTACACCGA

660 670 680 690 700
CACATCCACG AGGAGTGTTG CCAAGGAAAC TCGCTGGAGT GTCTGCAGGA
GTGTAGGTGC TCCTCACAAC GGTTCCTTTG AGCGACCTCA CAGACGTCCT

710 720 730 740 750
TGGGGAAAAA GTCATGACAT ATATATGTTC TCAACAAAAT ATTCTGTCAA
ACCCCTTTTT CAGTACTGTA TATATACAAG AGTTGTTTTA TAAGACAGTT

760 770 780 790 800
GCAAAATAGC AGAGTGCTGC AAATTACCCA TGATCCAACT AGGCTTCTGC
CGTTTTATCG TCTCACGACG TTTAATGGGT ACTAGGTTGA TCCGAAGACG

810 820 830 840 850
ATAATTCACG CAGAGAATGG CGTCAAACCT GAAGGCTTAT CTCTAAATCC
TATTAAGTGC GTCTCTTACC GCAGTTTGGA CTTCCGAATA GAGATTTAGG

860 870 880 890 900
AAGCCAGTTT TTGGGAGACA GAAATTTTGC CCAATTTTCT TCAGAGGAAA
TTCGGTCAAA AACCCTCTGT CTTTAAAACG GGTTAAAAGA AGTCTCCTTT

910 920 930 940 950
AAATCATGTT CATGGCAAGC TTTCTTCATG AATACTCAAG AACTCACCCC
TTTAGTACAA GTACCGTTCG AAAGAAGTAC TTATGAGTTC TTGAGTGGGG

960 970 980 990 1000
AACCTTCCTG TCTCAGTCAT TCTAAGAATT GCTAAAACGT ACCAGGAAAT
TTGGAAGGAC AGAGTCAGTA AGATTCTTAA CGATTTTGCA TGGTCCTTTA
 
1010 1020 1030 1040 1050
ATTGGAGAAG TGTTCCCAGT CTGGAAATCT ACCTGGATGT CAGGACAATC
TAACCTCTTC ACAAGGGTCA GACCTTTAGA TGGACCTACA GTCCTGTTAG

1060 1070 1080 1090 1100
TGGAAGAAGA ATTGCAGAAA CACATCGAGG AGAGCCAGGC ACTGTCCAAG
ACCTTCTTCT TAACGTCTTT GTGTAGCTCC TCTCGGTCCG TGACAGGTTC

1110 1120 1130 1140 1150
CAAAGCTGCG CTCTCTACCA GACCTTAGGA GACTACAAAT TACAAAATCT
GTTTCGACGC GAGAGATGGT CTGGAATCCT CTGATGTTTA ATGTTTTAGA

1160 1170 1180 1190 1200
GTTCCTTATT GGTTACACGA GGAAAGCCCC TCAGCTGACC TCAGCAGAGC
CAAGGAATAA CCAATGTGCT CCTTTCGGGG AGTCGACTGG AGTCGTCTCG

1210 1220 1230 1240 1250
TGATCGACCT CACCGGGAAG ATGGTGAGCA TTGCCTCCAC GTGCTGCCAG
ACTAGCTGGA GTGGCCCTTC TACCACTCGT AACGGAGGTG CACGACGGTC

1260 1270 1280 1290 1300
CTCAGCGAGG AGAAATGGTC CGGCTGTGGT GAGGGAATGG CCGACATTTT
GAGTCGCTCC TCTTTACCAG GCCGACACCA CTCCCTTACC GGCTGTAAAA

1310 1320 1330 1340 1350
CATTGGACAT TTGTGTATAA GGAATGAAGC AAGCCCTGTG AACTCTGGTA
GTAACCTGTA AACACATATT CCTTACTTCG TTCGGGACAC TTGAGACCAT

1360 1370 1380 1390 1400
TCAGCCACTG CTGCAACTCT TCGTATTCCA ACAGGAGGCT ATGCATCACC
AGTCGGTGAC GACGTTGAGA AGCATAAGGT TGTCCTCCGA TACGTAGTGG

1410 1420 1430 1440 1450
AGTTTTCTGA GGGATGAAAC CTATGCCCCT CCCCCATTCT CTGAGGATAA
TCAAAAGACT CCCTACTTTG GATACGGGGA GGGGGTAAGA GACTCCTATT

1460 1470 1480 1490 1500
ATTCATCTTC CACAAGGATC GTGCCAAGCT CGGCAAAGCC CTACAGACCA
TAAGTAGAAG GTGTTCCTAG CACGGTTCGA GCCGTTTCGG GATGTCTGGT

1510 1520 1530 1540 1550
TGAAACAAGA GCTTCTCATT AACCTGGTGA AGCAAAAGCC TGAACTGACA
ACTTTGTTCT CGAAGAGTAA TTGGACCACT TCGTTTTCGG ACTTGACTGT

1560 1570
GAGGAGCAGC TGGCGGCTGT CACTGCAG
CTCCTCGTCG ACCGCCGACA GTGACGTC
 The rat α-fetoprotein 3' end cDNA is as follows (540 bp's): 
10         20         30         40         50
GAGGGACTGG CCGACATTTA CATTGGACAC TTGTGTTTAA GACATGAGGC
CTCCCTGACC GGCTGTAAAT GTAACCTGTG AACACAAATT CTGTACTCCG

60         70         80         90         100
AAACCCTGTG AACTCCGGTA TCAACCACTG CTGCAGTTCC TCGTATTCCA
TTTGGGACAC TTGAGGCCAT AGTTGGTGAC GACGTCAAGG AGCATAAGGT
 
110 120 130 140 150
ACAGGAGGCT CTGCATCACC AGCTTTCTGA GGGACGAAAC CTACGTCCCT
TGTCCTCCGA GACGTAGTGG TCGAAAGACT CCCTGCTTTG GATGCAGGGA

160 170 180 190 200
CTACCATTCT CTGCGACAAA TTCATCTTCC ACAAGGAATC TGTGCCAAGC
GATGGTAAGA GACGCTGTTT AAGTAGAAGG TGTTCCTTAG ACACGGTTCG

210 220 230 240 250
TCAGGGCCGA GCACCACAGA CCATGAAACA AGAGCTTCTC ATTAACCTAG
AGTCCCGGCT CGTGGTGTCT GGTACTTTGT TCTCGAAGAG TAATTGGATC

260 270 280 290 300
TGAAACAAAA GCCTGAAATG ACAGAGGAGC AGCACGCGGC TGTCACTGCT
ACTTTGTTTT CGGACTTTAC TGTCTCCTCG TCGTGCGCCG ACAGTGACGA

310 320 330 340 350
GATTTCTCTG GCCTCTTGGA GAAGTGCTGC AAAGACCAGG ATCAGGAAGC
CTAAAGAGAC CGGAGAACCT CTTCACGACG TTTCTGGTCC TAGTCCTTCG

360 370 380 390 400
CTGTTTCGCA AAAGAGGTCC AAGTTGATTT CCAAACTCGT GAGGCTTTGG
GACAAAGCGT TTTCTCCAGG TTCAACTAAA GGTTTGAGCA CTCCGAAACC

410 420 430 440 450
GGGTTTAAAC ATCTCCAAGA GGAAGAAAGG ACAAAAAAAT GTGTCGACGC
CCCAAATTTG TAGAGGTTCT CCTTCTTTCC TGTTTTTTTA CACAGCTGCG

460 470 480 490 500
TTTGGTGTGA GCTTTTCGGT TTGATGGTAA CTGGTGGAGA CTTCCATGTG
AAACCACACT CGAAAAGCCA AACTACCATT GACCACCTCT GAAGGTACAC

510 520 530 540
GGATTTCTAT GCCTAAGGAA TAAAGACTTT TCAACTGTTA
CCTAAAGATA CGGATTCCTT ATTTCTGAAA AGTTGACAAT
 The homologous regions between both is as follows, (human: upper; rat: lower): 
***                    *              *   *          *        *
1367 CTCTTCGTATTCCAACAGGAGGCTATGCATCACCAG-TTTCTGAGGGATGAAACCTATG
90 CTC---GTATTCCAACAGGAGGCTCTGCATCACCAGCTTT-CTGAGGGACGAAACCTACG

*     **  *          **  **  *                    *  *
CCCCTC-CCCCATTCTCTGAGGA-TAAATTCATCTTCCACAAGGA-TC-GTGCCAAGCTC
TCCCTCTACC-ATTCTCTG-CGACAAA-TTCATCTTCCACAAGGAATCTGTGCCAAGCTC

*  ** * *  *  *                                *    *
-GG--CAAAGC-CCTACAGACCATGAAACAAGAGCTTCTCATTAACCTGGTGAAGCAAAA
AGGGCCGA-GCACC-ACAGACCATGAAACAAGAGCTTCTCATTAACCTAGTGAAACAAAA

       *               **
GCCTGAACTGACAGAGGAGCAGCTGGCGGCTGTCACTGC   1576
GCCTGAAATGACAGAGGAGCAGCACGCGGCTGTCACTGC    299

% = 85.845
P( 219, 188) = .000E+00     E = .000
 Restriction sites for both human and rat are as follows:

| | Human | | | | | | | |
|---|---|---|---|---|---|---|---|---|
| Mboll (GAAGA) | 108 | 261 | 328 | 1066 | 1069 | 1230 | | |
| Mboll (TCTTC) | 881 | 916 | 1361 | 1449 | | | | |
| MnII (CCTC) | 273 | 574 | 1190 | 1200 | 1219 | 1245 | 1439 | |
| MnII (GAGG) | 44 | 47 | 358 | 449 | 653 | 887 | 1070 | 1162 |
| | 1250 | 1274 | 1378 | 1402 | 1436 | 1544 | | |

| | Rat | | | | | | | |
|---|---|---|---|---|---|---|---|---|
| Mboll (GAAGA) | 435 | | | | | | | |
| Mboll (TCTTC) | 168 | | | | | | | |
| MnII (CCTC) | 100 | 159 | 323 | | | | | |
| MnII (GAGG) | 39 | 98 | 122 | 267 | 357 | 384 | 412 | |

It can be calculated that fragments containing a portion of the conserved sequence, *24, 34, 104* and *108* bp describe the human DNA. Fragments of *24, 59, 90* and *145* describe the rat DNA. While both sequences contain the *24* bp fragment, it is the *set* of fragments (taxonomic characters) that is of significance.

## Claims

1. A method of establishing the species of an unknown organism which comprises: determining the position of part or whole of an evolutionarily conserved sequence or sequences in genetic material (other than an evolutionarily conserved sequence occurring in ribosomal RNA information-containing nucleic acid) of said organism relative to a restriction endonuclease cleavage site in said genetic material, thereby to obtain an identifying genetic characterization of said unknown organism, comparing said characterization with information from at least two sets of identifying genetic characterizations derived from the same conserved sequences, each of said sets defining a known different organism species, and establishing the species of said unkown organism.

2. A method as claimed in Claim 1, wherein the genetic material is DNA.

3. A method as claimed in Claim 1, which comprises comparing the chromatographic pattern of restriction endonuclease digested DNA from said unknown organism, which digested DNA has been hybridized or reassociated with conserved DNA sequence information-containing nucleic acid from or derived from a probe oranism or from a consensus sequence, with equivalent chromatographic patterns of at least two known different organism species.

4. A method as claimed in Claim 3, wherein said conserved DNA information-containing nucleic acid is detectably labelled.

5. A method as claimed in Claim 4, wherein said conserved DNA information-containing nucleic acid is radiolabelled or metal labelled.

6. A method as claimed in Claim 3, 4 or 5, wherein said conserved DNA information-containing nucleic acid probe is an RNA probe.

7. A method as claimed in Claim 3, 4 or 5, wherein said conserved DNA information-containing nucleic acid probe is DNA complementary to RNA.

8. A method as claimed in Claim 3, 4 or 5, wherein said conserved DNA information-containing nucleic acid probe is DNA obtained by nick-translating or cloning DNA complementary to RNA.

9. A method as claimed in any one of Claims 1 to 3, wherein said unknown organism being characterized is a cell or cells of a strain in an *in vitro* culture.

10. A method as claimed in any one of claims 1 to 3, wherein said unknown organism being characterized and said probe organism are both from the same kingdom, subkingdom, division, subdivision, phylum, subphylum, class, subclass, order, family, tribe or genus.

11. A method as claimed in Claim 3, wherein said unknown organism being characterized and said probe organism are both eukaryotic.

12. A method as claimed in Claim 3, wherein said unknown organism being characterized and said probe organism are both prokaryotic.

13. A method as claimed in Claim 3, wherein said unknown organism being characterized is eukaryotic and said probe oranism is prokaryotic.

14. A method as claimed in Claim 3, wherein said unknown organism being characterized is prokaryotic and said probe organism is eukaryotic.

15. A method as claimed in Claim 12 or 14, wherein said prokaryotic organism being characterized is selectively being detected while in the presence of a eukaryotic organism.

16. A method as claimed in Claim 15, wherein said prokaryotic organism is a bacterium.

17. A method as claimed in Claim 11, wherein the DNA from said eukaryotic organism being characterized is nuclear DNA, and the conserved DNA information-containing nucleic acid from said eukaryotic probe organism is not derived from mitochondria or chloroplasts.

18. A method as claimed in Claim 11, wherein the DNA from said eukaryotic organism being characterized is mitochondrial DNA and the conserved DNA information-containing nucleic acid from said eukaryotic probe oranism is derived from mitochondria or chloroplasts.

19. A method as claimed in Claim 11, wherein the DNA from said eukaryotic organism being characterized is chloroplast DNA and the conserved DNA information-containing nucleic acid from said eukaryotic probe organism is derived from mitochondria or chloroplasts.

20. A method as claimed in Claim 13, wherein said DNA from said eukaryotic organism being characterized is derived from mitochondrial DNA.

21. A method as claimed in Claim 13, wherein said DNA from said eukaryotic organism being characterized is derived from chloroplast DNA.

22. A method as claimed in Claim 15, wherein said conserved DNA information-containing nucleic acid probe is derived from mitochondria or from chloroplasts.

23. A method of determining or establishing the species of an unknown bacterial strain present in a sample which comprises:
determining the position of part or whole of an evolutionarily conserved sequence or sequences in DNA (other than an evolutionarily conserved sequence occurring in ribosomal RNA information-containing nucleic acid) of said bacterium, relative to a restriction endonuclease cleavage site in said DNA, thereby to obtain an identifying genetic characterization of said unknown bacterium,
comparing said characterization with information from at least two sets of identifying genetic characterizations derived from the same conserved sequences, each of said sets defining a known different bacterial species, and establishing the species of said unknow, bacterial strain.

24. A method as claimed in Claim 23, which comprises comparing the chromatographic pattern of restriction-endonuclease digested DNA from said unknown bacterium, which digested DNA has been hybridized or reassociated with conserved DNA sequence information-containing nucleic acid from or derived from a probe bacterium or from a consensus sequence, with equivalent chromatographic patterns of known bacteria.

25. A method as claimed in Claim 23, wherein said unknown bacterium is present in a fermentation medium or in a secretion or excretion product.

26. A method as claimed in Claim 23, wherein said unknown bacterium is present in or associated with eukaryotic tissue.

27. A method as claimed in Claim 26, wherein said bacterium is present in or associated with animal or plant cells.

28. A method as claimed in Claim 26, wherein said bacterium is present in or associated with human cells, or associated with plant root cells.

29. A method as claimed in any one of Claims 24 to 28, wherein said conserved DNA information-containing nucleic acid from said probe bacterium is detectably labelled.

30. A method as claimed in Claim 29, wherein said label is a radiolabel or a metal label.

31. A method as claimed in Claim 29, wherein said nucleic acid from said probe bacterium is RNA.

32. A method as claimed in Claim 29, wherein said nucleic acid from said probe bacterium is complementary DNA to RNA.

33. A method as claimed in any one of Claims 24 to 28, wherein said unknown bacterium is pathogenic towards plants or animals.

34. A method as claimed in Claim 24, wherein said chromatographic patterns of known bacteria are present in a catalog containing patterns of restriction fragments conserved as a group among different strains of a species, but not conserved as a group among different species.

35. A kit comprising a carrier being compartmentalized to receive in close confinement therein one or more container means, wherein a first container means contains conserved genetic material sequence information-containing nucleic acid (other than ribosomal RNA information-containing nucleic acid) from or derived from a probe organism or from a consensus sequence; and wherein said kit also contains a catalog comprising patterns which consist essentially of conserved sequence containing restriction fragments which are conserved as a group among different strains of species but not conserved as a group among different species, wherein said catalog is a book, a computer tape, a computer disk or a computer memory.

36. A kit as claimed in Claim 35, wherein the genetic material is DNA.

37. A kit as claimed in Claim 35 or 36, which also comprises a second container means containing one or more restriction endonuclease enzymes.

38. A kit as claimed in Claim 36, wherein the conserved DNA sequence information-containing nucleic acid probe is detectably labelled.

39. A kit comprising a carrier being compartmentalized to receive in close confinement therein one or more container means, wherein a first container means contains conserved genetic material sequence information-containing nucleic acid (other than ribosomal RNA information-containing nucleic acid) from or derived from a probe organism or from a consensus sequence, said nucleic acid being in detectably labelled form; and
wherein said kit also comprises a second container means containing one or more restriction endonuclease enzymes selected so that the enzyme and probe will provide patterns which comprise conserved sequence-containing restriction fragments which are conserved as a group among different strains of a species but not conserved as a group among different species.

40. A kit as claimed in Claim 35, 37 or 39, wherein said conserved DNA sequence information-containing nucleic acid probe is RNA.

41. A kit as claimed in Claim 35, 37 or 39, wherein said conserved DNA sequence information-containing nucleic acid probe is DNA complementary to RNA.

42. A kit as claimed in Claim 40, which also comprises a container means containing one or more detectably labelled deoxynucleoside triphosphates.

43. A kit as claimed in Claim 35, 37, or 39, wherein said probe organism is a prokaryote.

44. A kit as claimed in Claim 35, 37 or 39, wherein said probe organism is a eukaryote.

45. A kit as claimed in Claim 43, wherein said prokaryote is a bacterium.

46. A kit as claimed in Claim 44, wherein said nucleic acid probe of said eukaryote is derived from an organelle thereof.

47. A kit as claimed in Claim 41, wherein said cDNA is labelled with ³²P, ¹⁴C or ³H.

48. A kit as claimed in Claim 41, wherein said cDNA is a faithful copy of the RNA it is derived from.

49. A kit as claimed in Claim 35, 37 or 39, which also comprises one or more container means containing viral nucleic acid probes.

50. A kit as claimed in Claim 39, wherein said catalogue is a book, a computer tape, a computer disk, or a computer memory.

51. A kit as claimed in Claim 35, 37 or 39, wherein said catalogue also includes viral chromatographic patterns.

## Patentansprüche

1. Verfahren zum Nachweis der Spezies eines unbekannten Organismus, welches umfaßt:
Bestimmung der Position eines Teils der oder der gesamten evolutionär konservierten Sequenz oder Sequenzen in genetischem Material (mit Ausnahme von in Nukleinsäuren vorkommenden evolutionär konservierten Sequenzen, die die Information für ribosomale RNA enthalten) eines Organismus in Relation zu einer Restriktionsendonuklease-Spaltungsstelle in dem genetischen Material, wodurch man eine identifizierende, genetische Charakterisierung des unbekannten Organismus erhält,
Vergleich dieser Charakterisierung mit der Information wenigstens zweier Sätze identifizierender genetischer Charakterisierungen, die aus den gleichen konservierten Sequenzen abgeleitet sind, wobei jeder Satz eine bekannte, verschiedene Organismenspezies definiert; und
Nachweis der Spezies des unbekannten Organismus.

2. Verfahren nach Anspruch 1, wobei es sich bei dem genetischen Material um DNA handelt.

3. Verfahren nach Anspruch 1, das den Vergleich des chromatographischen Musters der mit Restriktionsendonukleasen verdauten DNA des unbekannten Organismus, die mit der die konservierte DNA-Sequenzinformation enthaltenden Nukleinsäure, die aus dem Probenorganismus stammt oder von diesem oder aus einer Konsensussequenz abgeleitet ist, hybridisiert oder reassoziiert wurde, mit äquivalenten chromatographischen Mustern wenigstens zweier bekannter, verschiedener Organismenspezies umfaßt.

4. Verfahren nach Anspruch 3, wobei die Nukleinsäure, welche die konservierte DNA-Information enthält, nachweisbar markiert ist.

5. Verfahren nach Anspruch 4, wobei die Nukleinsäure, welche die konservierte DNA-Information enthält, radioaktiv oder durch Metalle markiert ist.

6. Verfahren nach Anspruch 3, 4 oder 5, wobei es sich bei der Nukleinsäurenprobe, welche die konservierte DNA-Information enthält, um eine RNA-Probe handelt.

7. Verfahren nach Anspruch 3, 4 oder 5, wobei es sich bei der Nukleinsäurenprobe, welche die konservierte DNA-Information enthält, um eine zur RNA komplementäre DNA handelt.

8. Verfahren nach Anspruch 3, 4 oder 5, wobei es sich bei der Nukleinsäurenprobe, welche die konservierte DNA-Information enthält, umeine DNA handelt, die durch Nick-Translatierung oder durch Klonierung einer zur RNA komplementären DNA erhalten wurde.

9. Verfahren nach einem der Ansprüche 1 bis 3, wobei es sich bei dem zu charakterisierenden unbekannten Organismus um eine Zelle oder Zellen eines Stammes in einer in vitro-Kultur handelt.

10. Verfahren nach einem der Ansprüche 1 bis 3, wobei der zu charakterisierende unbekannte Organismus und der Probenorganismus dem gleichen Reich, Unterreich, der gleichen Gruppe, Untergruppe, dem gleichen Phylum, Subphylum, der gleichen Klasse, Unterklasse, Ordnung, Familie, dem gleichen Stamm oder der gleichen Gattung angehören.

11. Verfahren nach Anspruch 3, wobei es sich bei dem zu charakterisierenden unbekannten Organismus und dem Probenorganismus jeweils um Eukaryonten handelt.

12. Verfahren nach Anspruch 3, wobei es sich bei dem zu charakterisierenden unbekannten Organismus und dem Probenorganismus jeweils um Prokaryonten handelt.

13. Verfahren nach Anspruch 3, wobei es sich bei dem zu charakterisierenden unbekannten Organismus um einen Eukaryonten und bei dem Probenorganismus um einen Prokaryonten handelt.

14. Verfahren nach Anspruch 3, wobei es sich bei dem zu charakterisierenden unbekannten Organismus um einen Prokaryonten und bei dem Probenorganismus um einen Eukaryonten handelt.

15. Verfahren nach Anspruch 12 oder 14, wobei der zu charakterisierende prokaryontische Organismus in Anwesenheit eines eukaryontischen Organismus selektiv erfaßt wird.

16. Verfahren nach Anspruch 15, wobei es sich bei dem prokaryontischen Organismus um ein Bakterium handelt.

17. Verfahren nach Anspruch 11, wobei es sich bei der DNA des zu charakterisierenden eukaryontischen Organismus um Kern-DNA und bei der Nukleinsäure des eukaryontischen Probenorganismus, welche die konservierte DNA-Information enthält, nicht um eine von Mitochondrien oder Chloroplasten stammende Nukleinsäure handelt.

18. Verfahren nach Anspruch 11, wobei es sich bei der DNA des zu charakterisierenden eukaryontischen Organismus um mitochondriale DNA und bei der Nukleinsäure des eukaryontischen Probenorganismus, welche die konservierte DNA-Information enthält, um eine von Mitochondrien oder Chloroplasten stammende Nukleinsäure handelt.

19. Verfahren nach Anspruch 11, wobei es sich bei der DNA des zu charakterisierenden eukaryontischen Organismus um chloroplastische DNA und bei der Nukleinsäure des eukaryontischen Probenorganismus, welche die konservierte DNA-Information enthält, um eine von Mitochondrien oder Chloroplasten stammende Nukleinsäure handelt.

20. Verfahren nach Anspruch 13, wobei die DNA des zu charakterisierenden eukaryontischen Organismus von mitochondrialer DNA stammt.

21. Verfahren nach Anspruch 13, wobei die DNA des zu charakterisierenden eukaryontischen Organismus von Chloroplasten-DNA stammt.

22. Verfahren nach Anspruch 15, wobei die Nukleinsäurenprobe, welche die konservierte DNA-Information enthält,von Mitochondrien oder von Chloroplasten stammt.

23. Verfahren zur Bestimmung oder zum Nachweis der Spezies eines in einer Probe vorhandenen unbekannten Bakterienstammes, welches umfaßt:
Bestimmung der Position eines Teils der oder der gesamten evolutionär konservierten Sequenz oder Sequenzen in der DNA (mit Ausnahme von evolutionär konservierten Sequenzen, die in die Information für ribosomale RNA enthaltenden Nukleinsäuren vorkommen) des Bakteriums in Relation zu eine Restriktionsendonuklease-Spaltungsstelle in der DNA, wodurch man eine identifizierende, genetische Charakterisierung des unbekannten Bakteriums erhält,
Vergleich dieser Charakterisierung mit der Information aus wenigstens zwei Sätzen identifizierender, genetischer Charakterisierungen, die von den gleichen konservierten Sequenzen abgeleitet sind, wobei jeder Satz eine bekannte, verschiedene Bakterienspezies definiert, und
Nachweis der Spezies des unbekannten Bakterienstamms.

24. Verfahren nach Anspruch 23, das den Vergleich des chromatographischen Musters der mit Restriktionsendonukleasen verdauten DNA des unbekannten Bakteriums, die mit der die konservierte DNA-Sequenzinformation enthaltenden Nukleinsäure, die von dem Probenbakterium stammt oder von diesem aus einer Konsensussequenz abgeleitet ist, hybridisiert oder reassoziiert wurden, mit äquivalenten, chromatographischen Mustern bekannter Bakterien umfaßt.

25. Verfahren nach Anspruch 23, wobei das unbekannte Bakterium in einem Fermentationsmedium oder in einem Sekretions- oder Exkretionsprodukt vorhanden ist.

26. Verfahren nach Anspruch 23, wobei das unbekannte Bakterium in einem eukaryontischen Gewebe vorhanden oder damit assoziiert ist.

27. Verfahren nach Anspruch 26, wobei das Bakterium in tierischen oder pflanzlichen Zellen vorhanden oder mit ihnen assoziiert ist.

28. Verfahren nach Anspruch 26, wobei das Bakterium in menschlichen Zellen vorhanden oder mit ihnen oder mit Pflanzenwurzelzellen assoziiert ist.

29. Verfahren nach einem der Ansprüche 24 bis 28, wobei die Nukleinsäure des Probenbakteriums, welche die konservierte DNA-Information enthält, nachweisbar markiert ist.

30. Verfahren nach Anspruch 29, wobei es sich bei der Markierung um eine radioaktive oder Metall-Markierung handelt.

31. Verfahren nach Anspruch 29, wobei es sich bei der Nukleinsäure des Probenbakteriums um RNA handelt.

32. Verfahren nach Anspruch 29, wobei es sich bei der Nukleinsäure des Probenbakteriums um eine zu RNA komplementäre DNA handelt.

33. Verfahren nach einem der Ansprüche 24 bis 28, wobei das unbekannte Bakterium pathogen gegenüber Pflanzen oder Tieren ist.

34. Verfahren nach Anspruch 24, wobei die chromatographischen Muster bekannter Bakterien in einem Verzeichnis vorhanden sind, das die Muster von Restriktionsfragmenten enthält, welche als Gruppe in verschiedenen Stämmen einer Spezies konserviert sind, aber nicht als Gruppe in verschiedenen Spezies.

35. Ausrüstung, umfassend einen Träger, der so unterteilt ist, daß in einer darin befindlichen engen Umgrenzung eine oder mehrere Aufbewahrungsvorrichtungen aufgenommen werden können, wobei die erste Aufbewahrungsvorrichtung eine die konservierte Sequenzinformation für genetisches Material enthaltende Nukleinsäure (mit Ausnahme einer die Information für ribosomale RNA enthaltenden Nukleinsäure) enthält, die aus dem Probenorganismus stammt oder von diesem oder einer Konsensussequenz abgeleitet ist; und die Ausrüstung weiterhin ein Verzeichnis enthält, das im wesentlichen Muster umfaßt, die aus Restriktionsfragmenten mit konservierter Sequenz bestehen, welche als Gruppe in verschiedenen Stämmen einer Spezies konserviert sind, aber nicht als Gruppe in verschiedenen Spezies, wobei das Verzeichnis ein Buch, ein Computerband, eine Computerdiskette oder ein Computerspeicher ist.

36. Ausrüstung nach Anspruch 35, wobei es sich bei dem genetischen Material um DNA handelt.

37. Ausrüstung nach Anspruch 35 oder 36, welche auch eine zweiten Aufbewahrungsvorrichtung umfaßt, die eine oder mehrere Restriktionsendonukleaseenzyme enthält.

38. Ausrüstung nach Anspruch 36, wobei die Nukleinsäurenprobe, welche die konservierte DNA-Sequenzinformation enthält, nachweisbar markiert ist.

39. Ausrüstung, umfassend einen Träger, der so unterteilt ist, daß in einer darin befindlichen engen Umgrenzung eine oder mehrere Aufbewahrungsvorrichtungen aufgenommen werden können, wobei die erste Aufbewahrungsvorrichtung eine die konservierte Sequenzinformation für genetisches Material enthaltende Nukleinsäure (mit Ausnahme einer die Information für ribosomale RNA enthaltenden Nukleinsäure) enthält, die aus dem Probenorganismus stammt oder von diesem oder einer Konsensussequenz abgeleitet ist, wobei die Nukleinsäure in einer nachweisbar markierten Form vorliegt; und wobei die Ausrüstung weiterhin auch eine zweite Aufbewahrungsvorrichtung umfaßt, die eine oder mehrere Restriktionsendonukleaseenzyme enthält, welche so ausgewählt sind, daß durch das Enzym und die Probe Muster bereitgestellt werden, die Restriktionsfragmente mit konservierter Sequenz umfassen, welche als Gruppe in verschiedenen Stämmen einer Spezies konserviert sind, aber nicht als Gruppe in verschiedenen Spezies.

40. Ausrüstung nach Anspruch 35, 37 oder 39, wobei es sich bei der Nukleinsäurenprobe, welche die konservierte DNA-Sequenzinformation enthält, um RNA handelt.

41. Ausrüstung nach Anspruch 35, 37 oder 39, wobei es sich bei der Nukleinsäurenprobe, welche die konservierte DNA-Sequenzinformation enthält, um eine zu RNA komplementäre DINA handelt.

42. Ausrüstung nach Anspruch 40, die auch eine Aufbewahrungsvorrichtung umfaßt, welche ein oder mehrere nachweisbar markierte Desoxynukleosidtriphosphate enthält.

43. Ausrüstung nach Anspruch 35, 37 oder 39, wobei es sich bei dem Probenorganismus um einen Prokaryonten handelt.

44. Ausrüstung nach Anspruch 35, 37 oder 39, wobei es sich bei dem Probenorganismus um einen Eukaryonten handelt.

45. Ausrüstung nach Anspruch 43, wobei es sich bei dem Prokaryonten um ein Bakterium handelt.

46. Ausrüstung nach Anspruch 44, wobei die Nukleinsäurenprobe des Eukaryonten von einer seiner Organellen stammt.

47. Ausrüstung nach Anspruch 41, wobei die cDNA mit ³²P, ¹⁴C oder ³H markiert ist.

48. Ausrüstung nach Anspruch 41, wobei die cDNA eine zuverlässige Kopie der RNA darstellt, von der sie abgeleitet ist.

49. Ausrüstung nach Anspruch 35, 37 oder 39, welche auch eine oder mehrere Aufbewahrungsvorrichtungen mit viralen Nukleinsäureproben umfaßt.

50. Ausrüstung nach Anspruch 39, wobei es sich bei dem Verzeichnis um ein Buch, ein Computerband, eine Computerdiskette oder einen Computerspeicher handelt.

51. Ausrüstung nach Anspruch 35, 37 oder 39, wobei das Verzeichnis auch virale chromatographische Muster enthält.

## Revendications

1. Méthode de détermination de l'espèce d'un organisme inconnu, qui comprend les étapes consistant à:
déterminer la position de tout ou partie d'une ou plusieurs séquences conservées lors de l'évolution, dans le matériel génétique (autre qu'une séquence conservée lors de l'évolution, présente dans l'acide nucléique contenant une information d'ARN ribosomal) dudit organisme par rapport à un site de clivage d'endonucléase de restriction dans ledit matériel génétique, en sorte d'arriver à une caractérisation génétique d'identification de l'organisme inconnu,
comparer cette caractérisation à l'information provenant d'au moins deux jeux de caractérisations génétiques d'identification dérivées des mêmes séquences conservées, chacun desdits jeux définissant une espèce connue différente d'organisme, et
déterminer l'espèce dudit organisme inconnu.

2. Méthode selon la revendication 1, caractérisée en ce que le matériel génétique est l'ADN.

3. Méthode selon la revendication 1, qui comprend les étapes consistant à comparer le tracé chromatographique de l'ADN digéré par l'endonucléase de restriction provenant dudit organisme inconnu, lequel ADN digéré a été hybridé ou réassocié à l'acide nucléique contenant une information de séquence d'ADN conservée provenant, ou dérivé d'un organisme sonde ou d'une séquence consensuelle, avec des tracés chromatographiques équivalents d'au moins deux espèces différentes, connues, d'organismes.

4. Méthode selon la revendication 3, caractérisée en ce que l'acide nucléique contenant une information d'ADN conservé est marqué de manière détectable.

5. Méthode selon la revendication 4, caractérisée en ce que l'acide nucléique contenant une information d'ADN conservé est marqué par un élément radioactif ou un métal.

6. Méthode selon la revendication 3, 4 ou 5 caractérisée en ce que la sonde d'acide nucléique contenant une information d'ADN conservé est une sonde d'ARN.

7. Méthode selon la revendication 3, 4 ou 5, caractérisée en ce que la sonde d'acide nucléique contenant un information d'ADN conservé est un ADN complémentaire de ARN.

8. Méthode selon la revendication 3, 4 ou 5, caractérisée en ce que ladite sonde d'acide nucléique contenant une information d'ADN conservé est un ADN obtenu par traduction crénée ou clonage d'ADN complémentaire à ARN.

9. Méthode selon l'une quelconque des revendications 1 à 3, caractérisée en ce que ledit organisme inconnu qui est en cours de caractérisation est une cellule ou des cellules d'une souche dans une culture in vitro.

10. Méthode selon l'une quelconque des revendications 1 à 3, caractérisée en ce que ledit organisme inconnu qui est en cours de caractérisation et ledit organisme sonde sont tous deux du même règne, sous-règne, division, sous-division, phylum, sous-phylum, classe, sous-classe, ordre, famille, tribus ou genre.

11. Méthode selon la revendication 3, caractérisée en ce que ledit organisme inconnu qui est en cours de caractérisation et ledit organisme sonde sont tous deux eucaryotiques.

12. Méthode selon la revendication 3, caractérisée en ce que ledit organisme inconnu qui est en cours de caractérisation et ledit organisme sonde sont tous deux procaryotiques.

13. Méthode selon la revendication 3, caractérisée en ce que l'organisme inconnu qui est en cours de caractérisation est eucaryotique et l'organisme sonde est procaryotique.

14. Méthode selon la revendication 3, caractérisée en ce que l'organisme inconnu qui est en cours de caractérisation est procaryotique et ledit organisme sonde est eucaryotique.

15. Méthode selon la revendication 12 ou 14, caractérisée en ce que l'organisme procaryotique qui est en cours de caractérisation est détecté sélectivement alors qu'il se trouve en présence d'un organisme eucaryotique.

16. Méthode selon la revendication 15, caractérisée en ce que ledit organisme procaryotique est une bactérie.

17. Méthode selon la revendication 11, caractérisée en ce que l'ADN provenant dudit organisme eucaryotique qui est en cours de caractérisation est un ADN nucléaire et l'acide nucléique contenant une information d'ADN conservé provenant dudit organisme sonde eucaryotique n'est pas dérivé de mitochondries ou de chloroplastes.

18. Méthode selon la revendication 11, caractérisée en ce que l'ADN de l'organisme eucaryotique qui est en cours de caractérisation est un ADN mitochondrial et l'acide nucléique contenant une information d'ADN conservé issu de l'organisme sonde eucaryotique est dérivé de mitochondries ou de chloroplastes.

19. Méthode selon la revendication 11, caractérisée en ce que l'ADN dudit organisme eucaryotique qui est en cours de caractérisation est un ADN chloroplastique et l'acide nucléique contenant une information d'ADN conservé provenant dudit organisme sonde eucaryotique est dérivé de mitochondries ou de chloroplastes.

20. Méthode selon la revendication 13, caractérisée en ce que l'ADN provenant dudit organisme eucaryotique qui est en cours de caractérisation est dérivé d'ADN mitochondrial.

21. Méthode selon la revendication 13, caractérisée en ce que l'ADN provenant dudit organisme eucaryotique qui est en cours de caractérisation est dérivé d'ADN chloroplastique.

22. Méthode selon la revendication 15, caractérisée en ce que la sonde d'acide nucléique contenant une information d'ADN conservé est dérivée de mitochondries ou de chloroplastes.

23. Méthode de détermination ou d'établissement de l'espèce d'une souche bactérienne inconnue présente dans un échantillon, qui comprend les étapes consistant à :
déterminer la position de tout ou partie d'une ou plusieurs séquences d'ADN conservées lors de l'évolution (autre qu'une séquence conservée lors de l'évolution, présente dans l'acide nucléique contenant une information d'ARN ribosomal) de ladite bactérie, relativement à un site de clivage par endonucléase de restriction dans ledit ADN, en sorte d'arriver à une caractérisation génétique d'identification de ladite bactérie inconnue;
comparer ladite caractérisation à l'information provenant d'au moins deux jeux de caractérisations génétiques d'identification issues des mêmes séquences conservées, chacun desdits deux jeux définissant une espèce bactérienne connue, différente, et
déterminer l'espèce de ladite souche bactérienne inconnue.

24. Méthode selon la revendication 23, qui comprend les étapes consistant à comparer le tracé chromatographique de l'ADN digéré par endonucléase de restriction provenant de ladite bactérie inconnue, cet ADN digéré ayant été hybridé ou réassocié à un acide nucléique contenant une information de séquence d'ADN conservée provenant ou dérivé d'une bactérie sonde ou d'une séquence consensuelle, à des tracés chromatographiques équivalents de bactéries connues.

25. Méthode selon la revendication 23, caractérisée en ce que ladite bactérie inconnue est présente dans un milieu de fermentation ou dans un produit de sécrétion ou d'excrétion.

26. Méthode selon la revendication 23, caractérisée en ce que ladite bactérie inconnue est présente dans un tissu eucaryotique ou est associée à un tel tissu.

27. Méthode selon la revendication 26, caractérisée en ce que ladite bactérie est présente dans des cellules animales ou végétales, ou est associée à celles-ci.

28. Méthode selon la revendication 26, caractérisée en ce que ladite bactérie est présente dans des cellules humaines ou est associée à de telles cellules, ou est associée à des cellules de racines de plantes.

29. Méthode selon l'une quelconque des revendications 24 à 28, caractérisée en ce que l'acide nucléique contenant une information d'ADN conservé qui provient de ladite bactérie sonde est marqué de manière détectable.

30. Méthode selon la revendication 29, caractérisée en ce que ledit marquage est un marquage par un élément radioactif ou un marquage par un métal.

31. Méthode selon la revendication 29, caractérisée en ce que ledit acide nucléique provenant de ladite bactérie sonde est un ARN.

32. Méthode selon la revendication 29, caractérisée en ce que ledit acide nucléique provenant de ladite bactérie sonde est un ADN complémentaire à ARN.

33. Méthode selon l'une quelconque des revendications 24 à 28, caractérisée en ce que ladite bactérie inconnue est pathogène vis-à-vis de plantes ou d'animaux.

34. Méthode selon la revendication 24, caractérisée en ce que les tracés chromatographiques de bactéries connues sont présents dans un catalogue contenant des tracés de fragments de restriction conservés en tant que groupe parmi différentes souches d'une espèce, mais non conservés en tant que groupe parmi différentes espèces.

35. Ensemble comprenant un support compartimenté pour recevoir, dans un espace restreint, un ou plusieurs récipients, caractérisé en ce qu'un premier récipient contient un acide nucléique contenant une information de séquence de matériel génétique conservée (autre que l'acide nucléique contenant une information d'ARN ribosomal) provenant ou dérivé d'un organisme sonde ou d'une séquence consensuelle; et caractérisé en ce que ledit ensemble contient également un catalogue comportant des tracés qui consistent essentiellement en séquences conservées contenant des fragments de restriction qui sont conservées sous la forme d'un groupe parmi différentes souches d'espèces, mais non conservées sous la forme d'un groupe parmi des espèces différentes, caractérisé en ce que ledit catalogue est un livre, une bande magnétique pour ordinateur, un disque pour ordinateur ou une mémoire d'ordinateur.

36. Ensemble selon la revendication 35, caractérisé en ce que le matériel génétique est l'ADN.

37. Ensemble selon la revendication 35 ou 36, qui comprend également un second récipient contenant une ou plusieurs enzymes endonucléases de restriction.

38. Ensemble selon la revendication 36, caractérisé en ce que la sonde d'acide nucléique contenant une information de séquence d'ADN conservée est marquée de manière détectable.

39. Ensemble comprenant un support compartimenté pour recevoir dans un espace restreint un ou plusieurs récipients, caractérisé en ce qu'un premier récipient contient un acide nucléique contenant une information de séquence de matériel génétique conservé (autre que l'acide nucléique contenant une information d'ARN ribosomal) issu ou dérivé d'un organisme sonde ou d'une séquence consensuelle, ledit acide nucléique se présentant sous une forme marquée de manière détectable, et
caractérisé en ce que ledit ensemble comprend également un second récipient contenant une ou plusieurs enzymes endonucléases de restriction, choisies pour que l'enzyme et la sonde fournissent des tracés qui comprennent des fragments de restriction contenant une séquence conservée, qui sont conservés sous la forme d'un groupe parmi différentes souches d'espèces, mais non conservés sous la forme d'un groupe parmi différentes espèces.

40. Ensemble selon la revendication 35, 37 ou 39 caractérisé en ce que ladite sonde d'acide nucléique contenant une information de séquence d'ADN conservé est ARN.

41. Ensemble selon la revendication 37, 37 ou 39, caractérisé en ce que ladite sonde d'acide nucléique contenant une information de séquence d'ADN conservée est un ADN complémentaire à ARN.

42. Ensemble selon la revendication 40, qui comprend également un récipient contenant un ou plusieurs déoxynucléosides-triphosphates marqués de manière détectable.

43. Ensemble selon la revendication 35, 37 ou 39, caractérisé en ce que ledit organisme sonde est un procaryote.

44. Ensemble selon la revendication 35, 37 ou 39, caractérisé en ce que ledit organisme sonde est un eucaryote.

45. Ensemble selon la revendication 43, caractérisé en ce que ledit procaryote est une bactérie.

46. Ensemble selon la revendication 44, caractérisé en ce que ladite sonde d'acide nucléique dudit eucaryote est dérivée d'un organite de celui-ci.

47. Ensemble selon la revendication 41, caractérisé en ce que ledit cADN est marqué par ³²P, ¹⁴C ou ³H.

48. Ensemble selon la revendication 41, caractérisé en ce que le cADN est une copie fidèle de l'ARN dont il dérive.

49. Ensemble selon la revendication 35, 37 ou 39, qui comprend également un ou plusieurs récipients contenant des sondes d'acides nucléiques viraux.

50. Ensemble selon la revendication 39, caractérisé en ce que le catalogue est un livre, une bande magnétique pour ordinateur, un disque pour ordinateur ou une mémoire d'ordinateur.

51. Ensemble selon la revendication 35, 37 ou 39, caractérisé en ce que ledit catalogue comprend également des tracés chromatographiques viraux.
